# NEW EUROPEAN PATENT SPECIFICATION

(11) **EP 1 641 436 B2**
(45) Date of publication and mention of the opposition decision: **10.05.2017**
(45) Mention of the grant of the patent: 04.09.2013
(21) Application number: 04777564.8
(22) Date of filing: 01.07.2004
(51) Int. Cl.: A61K 31/427, A61K 9/19, A61K 9/14, A61K 9/72, A61K 9/12

(54) **INHALABLE AZTREONAM LYSINATE FORMULATION FOR TREATMENT AND PREVENTION OF PULMONARY BACTERIAL INFECTIONS**
INHALIERBARE AZTREONAM-LYSINAT-FORMULIERUNG ZUR BEHANDLUNG UND PRÄVENTION VON BAKTERIELLEN INFEKTIONEN DER LUNGE
FORMULATION D'AZTREONAME LYSINATE INHALABLE, POUR LE TRAITEMENT ET LA PREVENTION D'INFECTIONS BACTERIENNENES PULMONAIRES

(30) Priority: 03.07.2003 US 613639; 30.06.2004 US 882985
(43) Date of publication of application: 05.04.2006
(60) Divisional application: 09015300.8
(73) Proprietor: Gilead Sciences, Inc., Foster City, CA 94404 (US)
(72) Inventor: MONTGOMERY, Alan, Bruce, Bellevue, WA 98030 (US); LINTZ, Frank-Christophe, 52222 Stolberg (DE); KELLER, Manfred, 81379 Munchen (DE)
(74) Representative: Sexton, Jane Helen
(86) International application number: PCT/US2004/021517
(87) International publication number: WO 2005/007132

(56) References cited:
- EP-A1- 0 297 580
- EP-A1- 1 417 958
- EP-A2- 2 158 901
- WO-A-02/051356
- WO-A-03/035030
- WO-A1-2004/052333
- WO-A1-2005/005424
- US-A- 3 984 403
- US-A- 4 826 973
- US-A- 4 946 838
- US-B2- 6 660 249
- Swarbrick, James: "Encyclopedia of Pharmaceutical Technology, 3rd Edition", 2007, Informa Healthcare, New York vol. 3, pages 2079-2081,
- RANADIVE, S.A. ET AL: 'Formation, isolation and identification of oligomers of aztreonam' EUROPEAN JOURNAL OF PHARMACEUTICAL SCIENCES no. 3, 1995, pages 281 - 291
- SMITH, J.J.: 'Developments in inhalation technology' DRUG FORMULATION & DELIVERY January 2000, pages 118 - 124
- Basic principles of particle size analysis (Beatop (Zhuhai) Instruments Limited-http://www.beatop.com/Articles/Basi c_principles_ofparticlesize_analysis.pdf)

## Description

### BACKGROUND OF THE INVENTION

### Field of the Invention

The current invention concerns a novel, safe, nonirritating and physiologically compatible inhalable aztreonam lysinate formulation suitable for treatment of pulmonary bacterial infections caused by gram negative bacteria, such as *Escherichia coli, Enterobacteria* species, *Klebsiella pneumoniae, K. oxytoca, Proteus mirabilis, Pseudomonas aeruginosa, Serratia marcescens, Haemophilus influenzae, Burkholderia cepacia, Stenotrophamonae maltophilia, Alcaligenes xylosoxidans.* In particular, the invention concerns the inhalable aztreonam lysinate formulation derived from α- or β-aztreonam suitable for treatment and prophylaxis of acute and chronic pulmonary bacterial infections, particularly those caused by gram-negative bacteria *Burkholderia cepacia, Stenotrophomonae maltophilia, Alcaligenes xylosoxidans,* and multidrug resistant *Pseudomonas aeruginosa* which are resistant to treatment with other antibiotics.

The inhalable aztreonam lysinate formulation is delivered as an aerosol or as an inhalable dry powder. For aerosolization, about 1 to about 250 mg of aztreonam lysinate is dissolved in about 1 to about 5 mL of saline or other aqueous solution having a pH between 4.5 and 7.5, delivered to the lung endobronchial space in an aerosol having mass medium average diameter particles predominantly between 1 to 5 µ using a nebulizer able to atomize the aztreonam lysinate solution into particles of required sizes. A combination of the novel formulation with the atomizing nebulizer permits about 50% delivery of the administered dose of aztreonam lysinate into airways. For delivery of dry inhalable powder, aztreonam lysinate is lyophilized, milled or spray dried to particle sizes between about 1 and 5 µ. Both the dry powder formulation or a reconstituted aztreonam lysinate solid for aerosolization have a long shelf-life and storage stability.

The invention further concerns a process for manufacturing and a large scale manufacturing α-aztreonam lysinate.

### Background and Related Disclosures

A wide variety of gram-negative bacteria cause severe pulmonary infections. Many of these bacteria are or become resistant to commonly used or specialty antibiotics and require treatment with new types of antibiotics. The pulmonary infections caused by gram-negative bacteria are particularly dangerous to patients who have decreased immunoprotective responses, such as, for example, cystic fibrosis and HIV patients, patients with bronchiectasis or those on mechanical ventilation.

Currently accepted therapy for severe bacterial respiratory tract infections, particularly for treatment of pneumonia in patients with underlying illnesses, includes treatment with various intravenous antibacterial agents, often used in two or three way combination. Most of these agents are not suitable, available or FDA approved for either oral or aerosol dosing. In some cases the efficacious systemic intravenous or oral dose requires doses which are borderline or outright toxic thus often preventing a use of perfectly good antibiotic for treatment of the pulmonary infections.

Thus it would be desirable to have available other modes of delivery routes of these antibiotics enabling a targeted delivery of smaller amounts of the antibiotic to endobronchial space of airways for treatment of these bacterial infections rather than administering the antibiotic systemically in large amounts.

Additionally, chronically ill patients are often affected with infections caused by bacteria which are largely resistant to commonly used antibiotics or, upon extended use of certain antibiotic, often develop strong resistance to such antibiotic. For example, chronic pulmonary colonization with *Pseudomonas aeruginosa* in patients with cystic fibrosis is a principal cause of their high mortality. When established, the chronic pulmonary infection is very difficult, if not impossible, to eradicate. More than 60% of cystic fibrosis patients are colonized with *Pseudomonas aeruginosa* bacterium strains which are largely resistant to regular and specialty antibiotics, such as piperacillin, ticarcillin, meropenem, netilmicin and only little sensitive to azlocillin, ciprofloxacin, timentin and ceftazidime. Many strains have also been shown to develop resistance to tobramycin and to colistin, if used continuously.

Often, after prolonged antibiotic therapy, a superinfection with organisms intrinsically resistant to oral, intravenous or inhaled antibiotics develops in patients with cystic fibrosis and other chronic pulmonary infections. The four most common drug resistant organisms are *Burkholderia cepacia, Stenotrophomonas maltophilia, Alcaligenes xylosoxidans,* and multidrug resistant *Pseudomonas aeruginosa.*

Cystic fibrosis patients infected with *Burkholderia cepacia* have an increased rate of mortality compared to those patients with *Pseudomonas aeruginosa* infections. In some cystic fibrosis patients, *Burkholderia cepacia* can cause a rapid fatality, as described, for example in Am. J. Respir. Crit. Care Med., 160: 5, 1572-7 (1999).

The high level of antibiotic resistance demonstrated by most strains of *Burkholderia cepacia* severely limits therapeutic options for its treatment (Clinics Chest Med., 19:473-86 (Sept. 1998)). Furthermore, unlike *Pseudomonas aeruginosa, Burkholderia cepacia* can cause epidemic spread among cystic fibrosis patients and therefore any patient infected with *Burkholderia cepacia* is usually isolated from other patients. This causes both additional expenses connected with caring for these patients and may also be psychologically devastating to the patient. Furthermore, most lung transplant centers will not perform a lung transplant on patients infected with *Burkholderia cepacia* (Clinics Chest Med., 19:473-86 (Sept. 1998)). Therefore, the *Burkholderia cepacia* infection is often viewed as a death sentence by patients with cystic fibrosis.

*Burkholderia cepacia* is usually resistant to the parenteral delivery of various antibiotics, including aztreonam lysinate, with showing only 5% of isolates to be sensitive to such treatment (Antimicrob. Agents Chemother., 34: 3, 487-8 (Mar. 1990)). Thus it would be advantageous to have available treatment for *Burkholderia cepacia* infections.

Other gram-negative bacteria intrinsically resistant to tobramycin can also complicate the care of a cystic fibrosis patient. These bacteria include *Stenotrophomonas maltophilia* and *Alcaligenes xylosoxidans.* Antibiotic therapy of these infections is usually also ineffective or leads to rapid emergence of drug resistance. Therefore, the successful treatment of all these infections requires that samples of these isolates are sent to a laboratory for complex antibiotic synergy determination of proper therapy for each individual patient (Ped. Pulmon., S17: 118-119 (1998)). It would, therefore, be also advantageous to provide a therapy for these rare but hard to treat bacterial infections.

Similarly, the development of P. aeruginosa infection with strains which are resistant to, that is which have a high minimal inhibitory concentration (MIC) to a majority of antibiotics including tobramycin, predicts declining lung function and also may disqualify the patient from consideration for lung transplant (Clinics Chest Med., 19:535-554 (Sept 1998)).

Existing antibiotic treatments for *Burkholderia cepacia, Stenotrophomonas maltophilia, Alcaligenes xylosoxidans,* and multidrug resistant *Pseudomonas aeruginosa* pulmonary infections are either ineffective, or lead to rapid emergence of drug resistance.

Aztreonam is a synthetic antibiotic which has a good biological activity against gram-negative bacteria and its arginine salt derived from the beta form has previously been used for intravenous treatment of bacterial infections. However, its use is severely limited due to its low efficacy requiring administration of very large intravenous doses between 1000 and 4000 mg a day in order to treat the infections caused by gram-negative bacteria and also by its salt derivatization which is not suitable for inhalation purposes. Although it would be an antibiotic of choice for complementary treatment of patients treated with tobramycin or other antibiotics, such treatment is not practical because of the high doses required and because of the complication encountered with the arginine salt.

Aztreonam is currently only available as an arginine salt. Arginine has been shown to be toxic to the lung and causes lung tissue irritation, inflammation, bronchospasm and cough and therefore is not suitable for delivery by aerosolization. Consequently, aztreonam arginine salt is not approved for inhalation use in the United States or elsewhere.
However, as the antibiotic for treatment of pulmonary bacterial infections caused by gram negative bacteria, aztreonam could become a drug of choice for such treatment, if it could be delivered by inhalation in therapeutically effective concentrations directly to the lungs and if the problems connected with the aztreonam arginine could be overcome by providing a different, safer and physiologically acceptable salt derivative.

The efficacious administration of aztreonam by inhalation is further complicated by a lack of safe, physiologically acceptable and stable formulations for use by inhalation. Aside from the physiologically acceptable salt, such formulation must meet several criteria, such as certain size range of inhalable particles, certain pH range and certain degree of salinity. When the aerosol contains a large number of particles with a mass medium average diameter (MMAD) larger than 5 µ, these are deposited in the upper airways decreasing the amount of antibiotic delivered to the site of infection in the endobronchial space of airways. Similarly, both highly acidic and alkaline or hypotonic or hypertonic conditions lead to respiratory complications, such as bronchospasm and cough, preventing inhalation of the drug.

It is, therefore, a primary object of this invention to provide an inhalation aztreonam formulation suitable to efficacious delivery of aztreonam into lung for treatment of pulmonary gram-negative infections, especially those caused by *Burkholderia cepacia, Stenotrophomonas maltophilia, Alcaligenes xylosoxidane,* and multidrug resistant *Pseudomonas aeruginosa* by providing a safe, physiologically acceptable and efficacious formulation for inhalation of a pure concentrated aztreonam lysinate salt, which formulation contains sufficient but not excessive concentration of about 75 mg/mL of aztreonam lysinate, which formulation can be efficiently aerosolized by nebulization using jet, ultrasonic or atomization nebulizers, into an aerosol having particle sizes within a range from 1 to 5 µ, or administered as a dry powder, both well tolerated by patients with cystic fibrosis and with impaired pulmonary function due to infections, inflammation or another underlying disease. It is another object of this invention to provide a process for manufacturing α-aztreonam lysinate from α-aztreonam on a large scale.

WO 03/035030 describes a kit for the preparation of a liquid pharmaceutical composition.

WO 02/051356 describes inhalable aztreonam for treatment and prevention of pulmonary bacterial infections.

### Summary

The present invention provides a process for preparing an aztreonam lysinate dry inhalable powder formulation consisting of aztreonam lysinate, the process comprising the steps of:
a) solubilizing the alpha form of aztreonam in water to form a slurry;
b) titrating an aqueous solution of lysine to form aztreonam lysinate in solution; and
c) drying the aztreonam lysinate from the solution by lyophilisation or spray drying; and wherein the aztreonam lysinate is milled, precipitated , spray dried or otherwise processed to a particle size between 1 to 5 microns.

A method is described for treatment of pulmonary infections caused by gram-negative bacteria by inhalation of aerosolized aztreonam lysinate.

A method is also described for treatment of pulmonary bacterial infections caused by gram-negative bacteria, said method comprising administration of an inhalable concentrated pure aztreonam lysinate in a dry powder form or as an aerosol containing from about 1 to about 250 mg of aztreonam lysinate, said aztreonam lysinate administered in an inhalable dry powder form or dissolved in from about 1 to about 5 mL of an aerosolable solution of pH between 4.5 and 7.5 containing from about 0.1 to about 0.9% of chloride or other anion to the lung endobronchial space of airways of a patient in need thereof by nebulization in an aerosol having a mass medium average diameter between about 1 and about 5 µ, once or several times a day typically up to a daily dose aztreonam lysinate of 350 mg a day but in no instance more than 750 mg a day.

A method is described for treatment of pulmonary bacterial infections caused by *Escherichia coli, Enterobacteria* species, *Klebsiella pneumoniae, K. oxytoca, Proteus mirabilis, Pseudomonas aeruginosa, Serratia marcescens, Haemophilus influenzae, Burkholderia cepacia, Stenotrophomonas maltophilia, Alcaligenes xylosoxidans,* and multidrug resistant *Pseudomonas aeruginosa* using an inhalable formulation of aztreonam lysinate delivered by inhalation to the endobronchial space of airways in a dry powder form or in an aerosol.

A formulation is described comprising from about 1 to about 250 mg of aztreonam lysinate in a diluted saline solution ranging from one tenth to a half normal saline or other aqueous solvent containing chloride or another anion, wherein said formulation has a pH between 5.5 and 7.0 and is delivered by aerosolization in about 1-5 mL of solution wherein aerosol has particles of the MMAD predominantly between 1 and 5 µ, wherein said formulation is nebulized using a jet, atomizing, electronic or ultrasonic nebulizer.

A dry powder formulation is described comprising from about 1 to 200 mg of α-aztreonam lysinate, wherein said formulation is lyophilized, milled, spray dried or precipitated into a fine powder having particles with the MMAD between 1 and 5 µ used for inhalation of the dry powder administered from one to four times per day not exceeding 750 mg per day.

A process is described for manufacturing of a bulk solution or a lyophilized pure α-aztreonam lysinate comprising about 75 mg of aztreonam per 1 mL of solvent.

The manufacturing process may be used for preparation of α-aztreonam lysinate from α-aztreonam without need for conversion of α-aztreonam to β-aztreonam, wherein the resulting α-aztreonam lysinate has a better stability, higher purity and better yield.

A two-part reconstitution system is described comprising an α-aztreonam lysinate in dry or lyophilized powder form and a diluent stored separately until use.

### Brief Description of Figures

Figure 1 is a scheme illustrating lyophilization parameters used for manufacturing a lyophilized α-aztreonam lysinate.
Figure 2 is a graph showing impurity analysis of bulk solutions and lyophilizates of α-aztreonam prepared according to manufacturing procedure I compared to aztreonam free acid expressed as an active pharmaceutical ingredient (API).
Figure 3 is a graph showing impurity analysis of bulk solutions and lyophilizates of β-aztreonam prepared according to manufacturing procedure I compared to aztreonam free acid expressed as an active pharmaceutical ingredient (API).
Figure 4 is a graph showing impurity analysis of bulk solutions and lyophilizates of α-aztreonam prepared according to manufacturing procedure II compared to aztreonam free acid expressed as an active pharmaceutical ingredient (API).
Figure 5 is a graph showing impurity analysis of bulk solutions and lyophilizates of β-aztreonam prepared according to manufacturing procedure II compared to aztreonam free acid expressed as an active pharmaceutical ingredient (API).
Figure 6 is a graph showing impurity analysis of bulk solutions, lyophilizates and reconstituted lyophilizates of α-aztreonam prepared according to the manufacturing procedure II compared to aztreonam free acid expressed as an active pharmaceutical ingredient (API).
Figure 7 shows aztreonam lysinate activity against *P. aeruginosa* in the absence (Figure 1A) or presence (Figure 1B) of hog gastric mucin. Aztreonam lysinate was added to yield a final concentration in the following multiples of the MIC: 0.0 (◆); 0.1 (□); 1.0 (■); and 10 (◊). Figure 1A, no added mucin; Figure 1B, 10% mucin added.
Figure 8 shows aztreonam lysinate activity against *P. aeruginosa* in the presence or absence of cystic fibrosis (CF) sputum. Aztreonam lysinate was added to yield a final concentration in the following multiples of the MIC: 0. 0 (◆); 0.1 (□); 1.0 (■); and 10 (◊). Figure 2A, no added sputum; Figure 2B, 1% sputum added.
Figure 9 shows tobramycin activity against *P. aeruginosa* in the presence or absence of added mucin. Tobramycin was added to yield a final concentration in the following multiples of the MIC: 0.0 (◆); 1.0 (□); and 10 (■). Figure 3A, no added mucin; Figure 3B, 10% mucin added.

### Definitions

As used herein:
"MMAD" means mass medium average diameter.
"Normal saline" means water solution containing 0.9% (w/v) NaCl.
"Diluted saline" means normal saline containing 0.9% (w/v) NaCl diluted into its lesser strength from about 0.1% to about 0.8%.
"Half normal saline" or "½ NS" means normal saline diluted to its half strength containing 0.45% (w/v) NaCl.
"Quarter normal saline" or "1/4 NS" means normal saline diluted to its quarter strength containing 0.225% (w/v) NaCl.
"One tenth normal saline" or "1/10 NS" means normal saline diluted to its one tenth strength containing 0.09% (w/v) NaCl.
"CF" means cystic fibrosis.
"Predominantly" means including at least 70% but preferably 90% of particle sizes between 1 and 5 µ.
"Physiologically acceptable solution" means a saline diluted to between 1/10 NS or 1 NS or another aqueous solution comprising from about 31 to about 154 mM of chloride or an equivalent concentration of bromine or iodine.
"Composition" means α- or β-aztreonam lysinate containing formulation additionally containing other components, such as excipients, diluents, isotonic solutions, buffers, etc.
"Formulation" means a specific composition formulated for specific use, such as for aerosolization of α- or β-aztreonam lysinate containing solution or nebulization of dry powder.
"Aztreonam lysinate composition", "aztreonam lysinate formulation" or "aztreonam lysinate" means a composition or formulation comprising an indicated amount of α-aztreonam or β-aztreonam lysinate salt. Thus, if for example, the dose of aztreonam lysinate comprises molar amount of aztreonam free base it contains 1.8 multiple molar amount of lysine. It is to be understood that both α- and β-aztreonam lysinates are intended to be covered by the term "aztreonam lysinate" unless specifically designated either as α- or β-aztreonam lysinate.
"Concentrated aztreonam lysinate, means α- or β-aztreonam lysinate concentrated into a form which permits dilution of, or more than, 75 mg of aztreonam in 1 ml of diluent.
"Alpha form of aztreonam" or "α-aztreonam" means an alpha sterochemical configuration of aztreonam. The alpha form of aztreonam is distinguishable from the beta, gamma and delta forms of aztreonam. Each form seems to have different chemical and physical properties, such as, for example, stability, crystallization point and diffraction curve. Differences between these two forms are described, for example in US patent 4,946,838. Alpha or beta aztreonam arginine salt are described in EP application 0 297 580 B1. Alpha, beta, gamma and delta forms of aztreonam and their chemical and physical properties are described in US patent 4,826,973.
"α-Aztreonam lysinate composition" or "α-aztreonam lysinate formulation" means a composition or formulation comprising an indicated amount of α-aztreonam lysinate salt. The α-aztreonam lysinate composition typically can comprise from about 50 mg to about 300 mg of anhydrous α-aztreonam and from about 35 mg to about 420 mg of lysine monohydrate per one milliliter of water for injection.
"Lyophilizate" means a dry residuum of α-aztreonam lysinate obtained by a process of lyophilization from a α-aztreonam lysinate bulk solution.

### DETAILED DESCRIPTION OF THE INVENTION

The current invention concerns a discovery that a specifically formulated inhalable aztreonam lysinate, particularly α-aztreonam lysinate, is efficacious for treatment of pulmonary infections caused by gram-negative bacteria.

Consequently, we describe here an inhalable composition and a method of treatment for pulmonary bacterial infections caused by *Escherichia coli, Enterobacter species, Klebsiella pneumoniae, Kleibsiella oxytoca, Proteus mirabilis, Pseudomonas aeruginosa, Serratia marcescens, Haemophilus influenzae,* including ampicillin-resistant and other penicillinases-producing strains and *Nitrobacter* species as well as for treatment of more rare bacteria, such as *Burkholderia cepacia, Stenotrophomonas maltophilia, Alcaligenes xylosoxidans,* and multidrug resistant *Pseudomonas aeruginosa.* The aztreonam lysinate is delivered to a patient's endobronchial space of airways by inhalation of a dry powder or an aerosol solution.

The method of treatment of pulmonary bacterial infections is especially suitable for treatment of patients with cystic fibrosis, bronchiectasis and patients with pneumonia assisted by ventilators, however it is also useful for treatment of other conditions that are complicated by infections caused by *Burkholderia cepacia, Stenotrophomonas maltophilia, Alcaligenes xylosoxidans,* and multidrug resistant *Pseudomonas aeruginosa* or other gram-negative bacteria.

A novel, efficacious, safe, nonirritating and physiologically compatible inhalable aztreonam lysinate composition is described suitable for treatment of pulmonary bacterial infections caused, by gram-negative bacteria particularly those which are resistant to treatment with other antibiotics. The inhalable formulation of aztreonam lysinate is suitable both for treatment and prophylaxis of acute and chronic pulmonary infections. The inhalable formulation is delivered as an aerosol or as an inhalable dry powder. For aerosolization, aztreonam lysinate is dissolved in a minimal volume of about 1 to about 5 mL of an aqueous solvent comprising chloride bromine or iodine ion, having a pH between 4.2 and 7.5, delivered to the endobronchial space in an aerosol having MMAD particles predominantly between 1 to 5 µ using a nebulizer able to aerosolize the aztreonam lysinate solution into particles of required sizes.

The current invention concerns finding that the aztreonam lysinate derived from the α-aztreonam, as compared to β-aztreonam, has better properties and is more suited for preparation of aztreonam lysinate salt for inhalable product. The use of the α-aztreonam for preparation of aztreonam lysinate provides demonstrable advantages in both manufacturing processes and results in the product with higher purity and better stability.

The process for preparation of a pure and stable α-aztreonam lysinate is novel in that until now, the α-aztreonam was described as an unstable intermediate suitable only for preparation of a stable β-aztreonam and its conversion to β-aztreonam was required for preparation of therapeutically useful aztreonam arginine.

It has been now discovered that aztreonam lysinate derived from the α-aztreonam, as compared to the β-aztreonam, prepared according to the process of the invention results in a more pure product. The use of α-aztreonam for preparation of α-aztreonam lysinate provides demonstrable advantages in manufacturing processes of a lyophilized or dry powder product and results in the product with higher purity and better stability.

### I. Aztreonam Lysinate for Inhalation

Aztreonam is a compound known under its chemical name (Z) -2-[[[(2-amino-4-thiazolyl)[[(2S,3S)-2-methyl-4-oxo-1-sulfo-3-azetidinyl] carbamoyl] methylene] amino] oxy]-2-methylpropionic acid.

Aztreonam is a known synthetic antibiotic with antibacterial activity against most gram-negative bacteria. Aztreonam is a monobactam and as such it has a unique monocyclic beta-lactam nucleus, and is therefore structurally different from other β-lactam antibiotics such as, for example penicillins, cephalosporins, or cephamycins. The sulfonic acid substituent in the 1-position of the ring activates the beta-lactam moiety. An aminothiazolyl oxime side chain in the 3-position and a methyl group in the 4-position confer the specific antibacterial spectrum and beta-lactamase stability.

Aztreonam is chemically known and available as alpha, beta, gamma and delta forms. Aztreonam arginine salt, known under its trade name AZACTAM® is derived from the beta form. AZACTAM® (aztreonam arginine for injection, USP) commercially available from DURA Pharmaceuticals, Inc., San Diego, California, contains aztreonam arginine salt as the active ingredient, and is currently FDA approved only for intramuscular or intravenous use (PDR, pg. 1159 (2001)).

### A. Disadvantages of Aztreonam Arginine Salt

Currently, the only commercially available aztreonam (AZACTAM®) comprises arginine that has been found to cause pulmonary inflammation when administered in an aerosol form to the lung of the human patients. When used as a potential aerosolized mucolytic agent in cystic fibrosis patients, it caused the lung inflammation, bronchospasm and irritation. A study described in Pediatrics, 55:96-100 (1975) identifies arginine as a substrate for production of nitric oxide radicals and recommends that arginine should not be used for inhalation in patients.

Nitric oxide radical reacts with the superoxide anion to form peronitrile, which is by itself toxic to the tissue and also may further react to form highly reactive and toxic hydroxyl radical. Since inflammation is a serious impairment for cystic fibrosis and all other diseases which this invention attempts to treat, use of arginine salt is not suitable as it would defeat this purpose and worsen rather than improve the patient conditions.

Arginine is also an important substrate for immune complex injury in the lung, as disclosed in PNAS, 14:6338-6342 (1991). Since the aerosolization concentrates high levels of the aerosolized drug in the lung as compared to dilution seen after intravenous administration, the aerosolization of the aztreonam arginine salt would be detrimental rather than advantageous for treatment of cystic fibrosis patients or patients suffering from pulmonary infections. Moreover, it would dilute and/or negate the effect of aztreonam.

Because it is formulated as arginine salt, aztreonam arginine is not suitable or approved for inhalation treatment and aerosol administration in the United States. Consequently, there is no known aztreonam or aztreonam lysinate containing formulation available for aerosol delivery to the endobronchial space of airways.

The only attempt to deliver aztreonam arginine intermittently to cystic fibrosis subjects is described in Spanish Annals on Pediatrics, 40: No.3 (1994) where such delivery was made in an open label trial in cystic fibrosis patients with intermittently administered 500 and 1000 mg of AZACTAM® USP arginine salt, twice a day for 21 days, using CR60 System 22 unit nebulizer. The intent of this study was to treat aztreonam sensitive *Pseudomonas aeruginosa* organisms, but not multidrug resistant *Pseudomonas aeruginosa.* No effort was made to treat *Burkholderia cepacia, Stenotrophomonas maltophilia,* infections caused by *Alcaligenes xylosoxidans* or other gram-negative bacteria.

Based on these observations, in order to provide a safe inhalable form of aztreonam, clearly, another aztreonam salt is needed for treatment of pulmonary infections by inhalation.
Aztreonam lysinate, particularly aztreonam lysinate derived from α-aztreonam, was found to be pharmacologically most acceptable for inhalation purposes without causing any undesirable reactions.

The preferred pharmaceutically acceptable aztreonam lysinate salt is derived from reaction of α- or β-aztreonam with lysine.

### B. α- and β-Aztreonam Lysinate

Previously, a preparation of aztreonam arginine and other salts, but not lysinate, involved almost exclusively the β-form of aztreonam. Alpha form of aztreonam was previously found to be unstable and unusable for preparation of therapeutic compositions. On the other hand, β-aztreonam was considered to be the stable form and if α-aztreonam was used at all it was first converted to β-aztreonam.

The US patent 4,946,838 presents conclusive evidence that up-to-date, α-aztreonam is considered to be an unstable form of aztreonam which must be converted to the β-aztreonam before it is used for preparation of any therapeutic product.

### a. Stability of α- and β-Aztreonam

Aztreonam can exist either in anhydrous amorphous and crystalline forms or in hydrated and solvated crystalline forms. The amorphous and hydrated forms interconvert under certain temperature and humidity conditions, and are both unstable. In the solid state, the anhydrous crystalline and solvated forms show good stability without interconversion. However, in the presence of excipients that release moisture, the anhydrous crystalline form rapidly decomposes to an extent dependent on moisture content and temperature.

Stability of the α- or β-aztreonam compound is determined by its loss at various temperatures. The prior art supports general belief that α-aztreonam is an unstable form of aztreonam. According to the prior art reports, after one week of storage, α-aztreonam shows approximately 1% loss at room temperature whereas at 80°C loss reaches 80%. In contrast, β-aztreonam, after 12 month storage at temperature between -20°C and 40°C, has less than 2% increase in impurity level and a decrease of only 3.0 to 3.5% in potency. Based on these results, β-aztreonam would seem to be a better and more stable compound.

However, α-aztreonam lysinate prepared according to the process of the invention was found to contain less impurities and have a better stability and is thus a more pure and stable compound.

### 2. Purity of α- and β-Aztreonam

For preparation of inhalable product, the active ingredient must be relatively pure or must be purified to remove impurities which do or would potentially cause bronchospasm, inflammation, irritation or cough. Consequently, the aztreonam for inhalation must be either prepared in a pure form or must be purified.

The type and degree of impurities in the inhalation formulation are important for and have a specific impact on the long term stability of the drug and on the shelf-life of the final product.

According to the prior art, the crystalline form of α- aztreonam is considered to be an unstable intermediary which must be converted to the stable β-aztreonam. Such conversion is achieved by recrystallization of α-aztreonam from an organic solvent, typically ethanol, into a very stable β-aztreonam. However, as a consequence of the recrystallization step, the recrystallized β-aztreonam typically contains between 1-2% of residual organic solvent and other impurities. A presence of these impurities makes the β-aztreonam less suitable for delivery by inhalation.

A re-crystallization process for preparation of β-aztreonam from α-aztreonam utilizes ethanol as a solvent. Using this process, however, leads to between 5000-10,000 ppm residual ethanol remaining in prepared β-aztreonam. FDA limits a permissible presence of ethanol to less than 5000 ppm. Moreover, over time, the presence of the residual ethanol in β-aztreonam leads to production of ethyl ester, an impurity which is undesirable and not present in the α-aztreonam.

In the process of developing this invention it was unexpectedly found that for preparation of a bulk solution or lyophilized form of aztreonam lysinate for aerosolization or nebulization, α-aztreonam, previously thought to be unstable, was actually a preferred form of a starting material for production of aztreonam lysinate. When compared to the β-aztreonam, the α-aztreonam lysinate was found to contain fewer impurities. Additionally, the process for its preparation is easier, faster and does not require the use of organic solvents leaving residual impurities.

The α-aztreonam lysinate prepared according to the process of the invention is a substantially pure compound not containing any substantial amount of impurities and contaminants.

### 3. Solubility of α- and β-aztreonams

For preparation of pharmaceutical inhalable products, solubility of the active ingredient, in this case aztreonam, in water or aqueous solvents is of importance.

The β-aztreonam is relatively insoluble in water and precipitates into a particular matter and clumps when mixed with water or other aqueous solvent during a dissolution step of the process for preparation of the lysine salt. Such precipitation and clumping leads to increase in impurity at least partially caused by opening of an open-chain nucleophilic ring. In the presence of moisture and under various temperature and humidity conditions, the opening of the open-chain nucleophilic ring increases unpredictably resulting in the compound's higher instability. Testing data shows the initial impurity levels generated by the reaction of the β-aztreonam with lysine salt is in the 1% range and close to the FDA permissible impurity level.

On the other hand, the impurity levels of α-aztreonam lysinate generated by a direct reaction of α-aztreonam with a lysine salt is less than 0.1%.

α-Aztreonam lysinate prepared according to the process of the invention is a pure compound readily dissolvable in acqueous solvents, such as saline or water.

### 4. Comparison of α- and β-Aztreonam Properties

Properties of α- and β-aztreonam and their suitability to be used in a process of manufacturing a bulk solution and lyophilized or dry powder α- and β-aztreonam lysinate for inhalation were determined and the following observations were made.

For preparation of aztreonam lysinate suitable for inhalation, it is important that a starting compound, α-or β-aztreonam, is readily soluble in water and has manageable physiological pH.

### a. β-Aztreonam

When studied vis-a-vis the above stated requirements, during manufacturing of bulk solutions for freeze drying and lyophilization, it was found that β-aztreonam cannot be suspended in water at intrinsic pH values because it immediately starts to polymerize and discolor. Independent of temperature, β-aztreonam at concentrations ranging from 50 to 150 mg/mL of water gels within 15 minutes. This eliminates possibility of using reactions whereby a lysine solution would be added to β-aztreonam. The only other possibility for preparation of β-aztreonam lysinate is, therefore, to add β-aztreonam to the lysine solution.

A process of adding β-aztreonam to the lysine solution requires a treatment of β-aztreonam solution with high sheer equipment to achieve rapid dissolution of β-aztreonam before its addition to the lysine solution. In any case, the high pH (around 10) of the lysine solution causes significantly increased formation of certain impurity, herein called impurity B, in the β-aztreonam within short period of time.

Although the above method resulted in manufacturing of β-aztreonam lysinate salt with relatively high but acceptable impurity level, the procedure for its preparation was found to be doable but not overly practical for production of β-aztreonam lysinate on a commercial scale, as it involves a rapid addition of β-aztreonam to the lysine solution. Both solutions, that is β-aztreonam and lysine solution, independently, need to be handled with a special equipment and care. β-aztreonam solution requires a high-shear mixer for mixing β-aztreonam and maintaining it in the solution as well as a special dosing equipment for its rapid addition to the lysine solution.

### b. α-Aztreonam

On the other hand, α-aztreonam can be easily suspended in water to form a homogeneous slurry, having an acidic pH which enhances its stability. Salt formation can thus be easily performed by adding a lysine solution to the α-aztreonam slurry. This step permits monitoring of the pH during salt formation and the formulation can easily be kept within a pH lower than 6, that is at a pH range offering adequate stability of the formed α-aztreonam lysinate. There is no need for high sheer mixer, dosing or any other equipment.

### c. Distinctions between α- and β-Aztreonam

The important distinction between α- and β-aztreonam is their solubility in water. α-Aztreonam is water soluble at a slightly acidic pH, β-aztreonam is not water soluble and cannot be dissolved at intrinsic pH values.

β-Aztreonam polymerizes, clumps and solidifies when mixed with water without intervention of a high shear mixing equipment. α-aztreonam is readily soluble in water and forms a slurry.

Additionally, β-aztreonam must be added to the lysine solution whereas lysine solution can be added to the α-aztreonam slurry.

Mixing and dosing equipment and a rapid mixing is needed to achieve β-aztreonam dissolution in water and for adding it to the lysine solution. No equipment is needed for dissolution of α-aztreonam in water as the α-aztreonam is readily soluble at the pH lower than 6 and lysine solution can be advantageously added to the α-aztreonam without any substantial pH change.

### d. Evaluation of Manufacturing Options

In order to evaluate the manufacturing options for preparation of aztreonam lysinate for inhalation, α-aztreonam and β-aztreonam were used as starting materials to manufacture bulk solutions, dry powder and lyophilizates. The procedures used to manufacture the bulk solutions represent the two obvious possibilities: 1) addition to α- or β-aztreonam to a lysine solution; and 2) addition of the lysine solution to β-aztreonam solution or to α-aztreonam slurry.

The analytical results described below indicate that the addition of a lysine solution to the crystalline form of α-aztreonam offers the most versatile handling options for large scale manufacturing.

### C. Development of Manufacturing Process for Preparation of Aztreonam Lysinate

In pursuance of the original aim to develop a workable and practical process for large scale manufacturing of aztreonam lysinate for inhalation, both α-and β-aztreonam lysinate were investigated.

### 1. Materials

All materials used are commercially available. α-aztreonam was obtained from Butical SpA. β-Aztreonam was obtained from Teva Corp., Israel. Lysine monohydrate was purchased from Merck KGaA. Water was purified by reverse osmosis.

### 2. Methods

There were two manufacturing processes used for development and optimization of the manufacturing process for preparation of a bulk solution and ultimately for preparation of a lyophilized aztreonam lysinate.

The two processes are identified as a manufacturing Process I wherein the appropriate amounts of α- or β-aztreonam were added to the lysine solution, and a manufacturing Process II wherein the lysine solution was added to the α- or β-aztreonam.

It was previously determined that for an efficacious inhalable aztreonam product, the bulk solution need to contain about 75 mg/mL of aztreonam lysinate. Consequently, the necessary amounts of aztreonam, lysine and water were calculated such as to reach an optimal aztreonam concentration of 75.0 mg/mL in the bulk solution and an aztreonam to lysine ratio of 1.4:1. The bulk solution need to have pH in around pH 4.8. The batch sizes of the bulk solutions prepared for testing were 200 mL.

Typically, the bulk solution manufacturing and salt formation process were performed within a double jacket glass flask in order to control the temperature throughout the process. The bulk solutions were manufactured according to the following procedures.

### 3. Manufacturing Processes

### a) Manufacturing Process I

Manufacturing process I comprises four steps.
Step 1) A required amount of lysine-monohydrate was weighed and dissolved in an appropriate amount of purified water generated by reverse osmosis using a magnetic stirrer and subsequently filtered through 0.22 µm membrane filter at room temperature (20° C ± 2°C).
Step 2) After complete dissolution of lysine-monohydrate, the lysine solution was brought to 2-8°C temperature using the refrigerated cooler connected to the double jacket glass flask. The temperature was controlled with a temperature probe in the solution.
Step 3) A necessary amount of α- or β-aztreonam to reach concentration 75 mg/mL was added to the lysine solution under constant stirring and mixing. Mixing was performed by magnetic stirrer in combination with an Ultra Turrax (11,000 rpm, 30 seconds). Preliminary experiments had shown that for β-aztreonam the use of a magnetic stirrer alone leads to unsatisfactory results. This is due to the fact that the low mixing intensity of the magnetic stirrer cannot prevent particles of β-aztreonam to adhere to the walls of the flask or to form agglomerates.
Step 4) After addition of the α- or β-aztreonam, mixing was continued until total dissolution occurred (yellowish solution free of particulate matter). During salt conversion, the temperature and pH of the formulation were constantly monitored.

### b) Manufacturing Process II

Manufacturing process II comprises three steps.
Step 1) 50% of the calculated amount of purified water at 2-8°C was used to form a slurry with α- or β-aztreonam using magnetic stirrer.
Step 2) The necessary amount of lysine-monohydrate was dissolved in the remaining water having the same temperature as the aztreonam slurry and slowly added to the slurry under constant stirring. The addition rate was such that the pH remained lower than pH 6 during the salt conversion.
Step 3) Stirring was continued until a total dissolution of aztreonam occurred. The total dissolution of the aztreonam was identified by a yellow solution free of particulate matter.

### c) pH and Temperature Measurements

Since the pH values of dissolved α- and β-aztreonam in water are different, pH and temperature were followed during evaluation of both processes.

Temperature and pH values of test solutions were assessed with an electronic pH-meter equipped with a glass electrode. Before each set of measurements, the pH-meter was calibrated using appropriate standards with pH values of 4.0 and 10.0. Measurements were carried out at the selected temperature for every given experiment. During salt formation, the pH and temperature of the bulk were automatically recorded every 5 seconds.

The bulk solutions of α- or β-aztreonam lysinate were manufactured at different temperatures ranging from 2°C to 20°C in order to determine the temperature dependency of the pH of a 75 mg/ml α-aztreonam lysinate solution.

### d) Lyophilization

Bulk solutions of α- and β-aztreonam prepared according to manufacturing process I or II were lyophilized and a degree of impurities in each bulk solution and lyophilizate were determined. To that end, one ml aliquots of the bulk solutions were dispensed into glass lyophilization vials (1.0 mL bulk solution per vial) and lyophilized according to the following conditions.

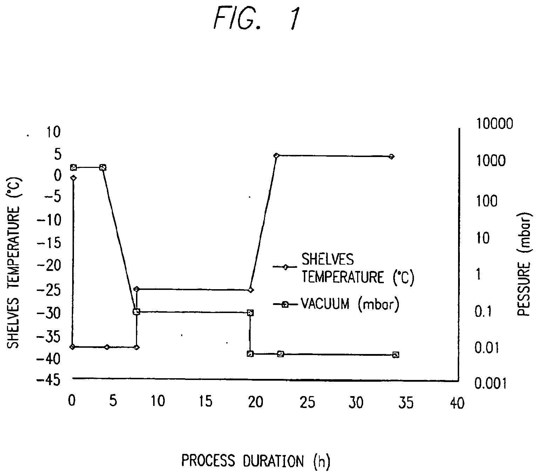

The shelves of the lyophilizer were prechilled to 0°C before the start of operations to allow a rapid freezing of the bulk solution in the vials. Lyophilization vials containing the bulk solution were placed on the prechilled shelves of the lyophilizer. Lyophilizer interior was then chilled to -38°C at which temperature samples of the bulk solutions in vials were frozen, the temperature of the lyophilizer was maintained at -38°C and vacuum was adjusted to 0.08 mbar at constant rate within 3 hours. Lyophilizer temperature was then raised to -25°C within 15 minutes and maintained for about 11 hours. Vacuum was then adjusted to 0.047 mbar. Temperature was increased to +5°C within 3 hours and maintained for 12 hours. After 12 hours at +5°C, vacuum was removed, vials were closed and crimped. The process parameters are depicted in Figure 1, which shows time progress, temperature of shelves and the vacuum pressure used for lyophilization.

### e. Impurity Analysis

The impurity profiles of the manufactured bulk solutions and lyophilizates were determined by high pressure liquid chromatography (HPLC).

HPLC was carried out using mobile phase A and B. The mobile phase A comprised ammonia formate buffer (pH 3.0) and methanol (94:6). The mobile phase B comprised ammonia formate buffer (pH 3.0) and methanol in ratio 55:45. Reference substances used as standard for detection of impurities were aztreonam; open ring aztreonam; aztreonam E isomer; (Z)-2-(aminothiazole-4-yl)-2-(t-butoxycarbonyl)isopropacyimino acetic acid (ATBA); 2-mercapto-benzothiazole (MBTA) and t-butyl-aztreonam (t-butyl ATR). Standards were prepared as impurity stock solutions and run in parallel with the aztreonam lysinate sample.

HPLC conditions were: 150 x 3 mm column (4 µm); column temperature 30°C; sample temperature 10°C; flow rate 0.6 ml/min; injection volume 40 µl, detection wavelength 270 nm and run time 40 minutes.

Both the aztreonam bulk solutions or lyophilized samples containing different concentrations of aztreonam or aztreonam lysinate were investigated for presence of impurities.

Impurities and degradation products of α- and β-aztreonam were separated by RP-HPLC and detected by UV detection at 270 nm. Quantification was carried out and expressed as area% of all integrated peaks greater than 0.1%.

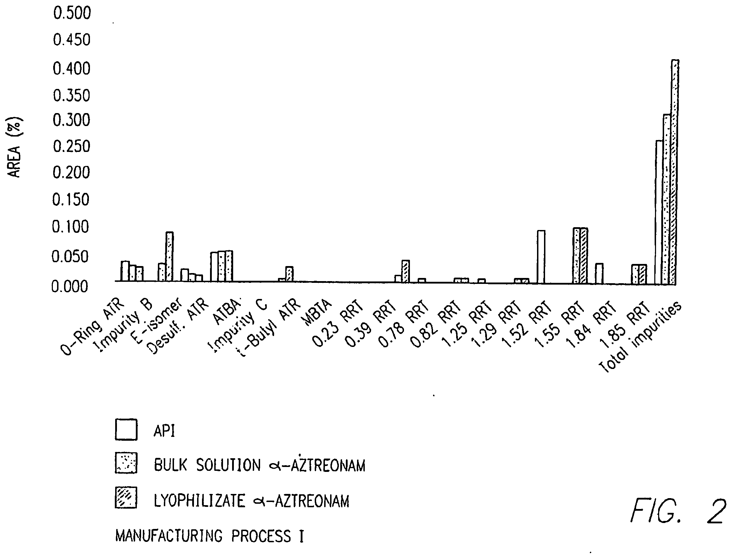

Impurities profiles for both α- and β-aztreonam lysinates in a graphical form are shown in Figures 2-5.

### 4. Evaluation of the Two Manufacturing Processes

The two aztreonam forms were evaluated by the two manufacturing processes for their conversion to the lysinate salt and the effect of the selected process on purity levels and stability.

### a. Salt Conversion

Conversion of α- or β-aztreonam into its lysine salt, as described above, depends on the starting form of aztreonam as well as on the manufacturing process used.

α-aztreonam was found to be compatible with both manufacturing processes. This was due to the fact that α-aztreonam can be suspended in water without solidifying, a problem observed with β-aztreonam. Because β-aztreonam solidifies nearly immediately when added to water, the salt conversion upon addition of lysine solution took longer and relatively high degree of degradation of β-aztreonam was observed. Table 1 summarizes the findings regarding the salt conversion step.

**Table 1**

| Salt Conversion | | |
|---|---|---|
| | Manufacturing Process I | Manufacturing Process II |
| α-Aztreonam lysinate | • No unusual occurrence during salt conversion | • No unusual occurrence during salt conversion |
| | • Yellowish, slightly opalescent solution | • Yellowish, slightly opalescent solution |
| β-Aztreonam lysinate | • No unusual occurrence during salt conversion | • ATR-slurry partly solidified (within 30 secs) before lysine addition. Therefore slow salt conversion upon addition of lysine solution |
| | • Clear, yellowish solution | |
| | | • Clear, pinkish solution |

ATR is used as an abbreviation for aztreonam.

Results show that when using α-aztreonam, the more simple manufacturing process II can be used for the lysine salt production without any consequences. When the starting aztreonam is β-aztreonam, the manufacturing process I needs to be used for the salt conversion. When used for preparation of β-aztreonam lysinate, the process II results in impure and unstable aztreonam lysinate. On the other hand, the process I requires more complicated and expensive equipment and because of the presence of impurities, it requires also more extensive validation work, especially when aztreonam lysinate is prepared on a large production scale.

### b. Aztreonam Content in Bulk Solutions

The aztreonam contents found in the bulk solutions manufactured by the processes I and II with starting concentration of aztreonam of 75.0 mg/mL, immediately after manufacturing are summarized in Table 2.

**Table 2**

| Aztreonam Content of Bulk Solutions | | |
|---|---|---|
| | Aztreonam Content (mg/mL) | |
| | Bulk Manufacturing Process I | Bulk Manufacturing Process II |
| α-Aztreonam lysinate | 74.7 | 74.7 |
| β-Aztreonam lysinate | 74.0 | 62.9 |

Aztreonam content is mean of 2 injections per solution.

As seen in Table 2, there is only small loss observed in both aztreonam forms when the process I is used. When the process II is used, the same amount of α-aztreonam is present in the bulk solution as in the bulk solution prepared by the process I. However, the results seen in Table 2 indicate that there is a significant degradation of β-aztreonam when following the manufacturing process II. This is at least partly due to the solidifying of β-aztreonam observed during manufacturing process II where upon the dissolution in water the total surface area of β-aztreonam is reduced and the salt conversion and dissolution is slower compared to α-aztreonam under the same conditions. Degradation reactions of β-aztreonam which are pH dependent occur. This phenomenon is not observed with α-aztreonam that, as pointed out above, is easily dissolved in water at pH under pH 6.0. As described above, α-aztreonam dissolved in water forms a homogeneous slurry having a large surface area.

### c. Lyophilization

Lyophilization conditions used for preparation of lyophilized α-aztreonam and β-aztreonam were suitable for both forms of aztreonam.

No significant differences between the two aztreonam forms were observed during freeze drying operations. All freeze-dried residues looked homogeneous without any wet, shrinked or sintered areas observed at the bottom of the lyophilization vials. Furthermore, no aztreonam particles were found on the vial wall, indicating that adequate temperature, pressure and ramping rates were used during freeze drying. The dissolution rate for both lyophilized aztreonams was very good with a reconstitution of both freeze-dried residues occurring in less than 1 sec after addition of solvent (0.17% saline/1.0 ml).

The selected freeze drying cycle lead to lyophilizates with generally low water contents of between 0.3-0.5%. As the water content in the final freeze-dried aztreonam lysinate could be raised to around 2.0% without stability problems, the cycle duration could be further shortened to approximately 30 hours.

Table 3 summarizes the water contents of the two lyophilizates.

**Table 3**

| Water Content in the Lyophilizates | | |
|---|---|---|
| | Water Content in the Lyophilizates (%) | |
| | Bulk Manufacturing Process I | Bulk Manufacturing Process II |
| α-Aztreonam lysinate lyophilizates | 0.5% | 0.4% |
| β-Aztreonam lysinate lyophilizates | 0.3% | 0.3% |

Water content in the lyophilizates is mean of 3 vials per batch.

Water content in both aztreonam lysinate lyophilizates is well under the acceptable level of water (2.0%) which could affect the aztreonam lysinate stability.

The aztreonam content in the lyophilization vials after lyophilization of both forms of aztreonam lysinates prepared by the processes I and II is summarized in Table 4.

**Table 4**

| Aztreonam Content per Vial After Lyophilization | | |
|---|---|---|
| | Aztreonam Content/Vial (mg) | |
| | Bulk Manufacturing Process I | Bulk Manufacturing Process II |
| α-Aztreonam lysinate lyophilizates | 73.8 | 74.0 |
| β-Aztreonam lysinate lyophilizates | 73.1 | 60.9 |

Aztreonam content is mean of 3 vials.

As expected from the bulk solution results seen in Table 2, lyophilizates made of β-aztreonam following the manufacturing process II contain much less than 75.0 mg aztreonam. There is a significant loss of approximately 12% of β-aztreonam during manufacturing process II. The results obtained from the lyophilization process indicate, that no significant loss of either of the aztreonams occurs during the freeze drying operation. The small decrease in amount of aztreonams observed in Table 4 compared to amounts seen in Table 2 is attributed to dispensing operations and to the reconstitution of the cakes prior to analysis. Therefore, the lyophilization cycle and its ramping rates can be regarded as valid for the bulk solution/vial combination.

### d. Analysis of Total Impurities of α- and β-Aztreonam

Analysis of total impurities in the two aztreonam forms is shown in Figures 2-5 and Table 5.

Figure 2 shows impurity analysis of bulk solutions and lyophilizates of α-aztreonam lysinate manufactured by manufacturing process I compared to the aztreonam active pharmaceutical ingredient (API). As seen in the last column of Figure 2, the total impurities observed in α-aztreonam lysinate in the API were 0.280%, in the bulk solution were 0.332% and total impurities in lyophilizates reaching 0.436% of the area.

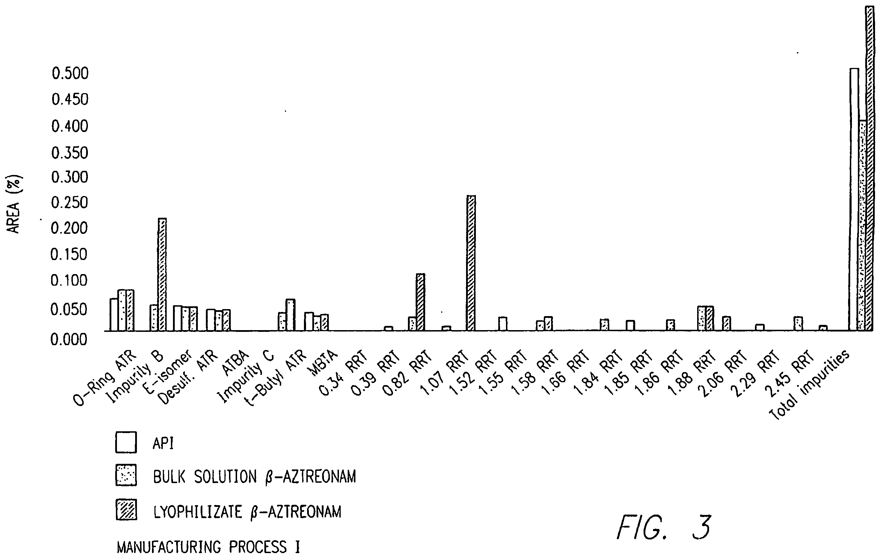

Figure 3 shows impurity analysis of bulk solution and lyophilizates of β-aztreonam lysinate manufactured by manufacturing process I compared to API. As seen in the last column of Figure 3, total impurities in the API were 0.370%, in the β-aztreonam bulk solution were 0.295% of area and the impurities found in lyophilizates were 0.457% of the area.

Observed levels of impurities in the α- and β-aztreonam were approximately the same for both aztreonam forms.

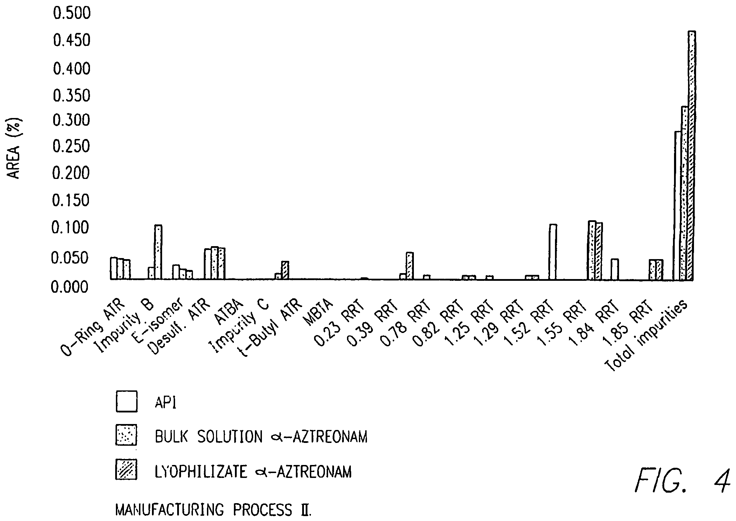

Figure 4 shows impurity analysis of the bulk solutions and lyophilizates of α-aztreonam prepared by manufacturing process II. As seen in Figure 4, the total impurities present in the API were 0.280%, in the bulk solution were 0.328% of the area and total impurities in lyophilizates were 0.471% of the area.

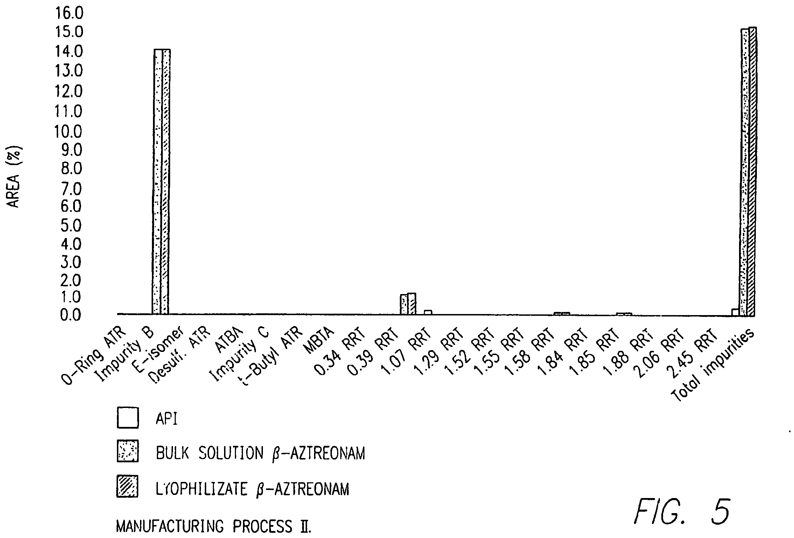

Figure 5 shows analysis of the bulk solution and lyophilizates of β-aztreonam compared to API prepared by the manufacturing process II. As seen in Figure 5, the impurities detected in API were 0.370%, which roughly corresponds to levels detected in Figures 2, 3, and 4. Levels of impurities detected in the β-aztreonam bulk solutions and lyophilizates were extremely high. Both the bulk solution and the lyophilizate of β-aztreonam produced by manufacturing process II contained 15,812% of the area in the bulk solution and 15,867% of the area in the lyophilizates.

Compared to the total impurities observed in the α-aztreonam bulk solution and lyophilizate, the levels of impurities in the β-aztreonam were almost 34 times higher in lyophilizates and 48 times higher in the bulk solutions.

Table 5 summarizes the obtained data for levels of impurities present in the bulk solutions and lyophilizates for α-and β-aztreonam lysinate.

**Table 5**

| Total Impurities | | | | |
|---|---|---|---|---|
| | Bulk Solutions | | Lyophilizates | |
| | α-aztreonam lysinate | β-aztreonam lysinate | α-aztreonam lysinate | β-aztreonam lysinate |
| Manufacturing Procedure I | 0.332% | 0.295% | 0.436% | 0.457% |
| Manufacturing Procedure II | 0.328% | 15.912% | 0.471% | 15.867% |

Impurities are expressed as a percentage of the area.

When using α-aztreonam as active pharmaceutical ingredient (API), no significant differences can be found between manufacturing processes I and II. The bulk solutions have an impurity level of around 0.33 area %, which represents an increase of around 0.04-0.05% versus the API. The lyophilizates have total impurities of around 0.45 area %, which represents an increase of around 0.08-0.12% versus API. It is important to emphasize that most single impurities found after manufacturing α-aztreonam lysinate using the described manufacturing processes I and II are below the level of 0.1% and consequently, both processes may be conveniently used for manufacturing α-aztreonam lysinate. When using the 0.1% FDA reporting limit, the α-aztreonam bulk solutions and lyophilizates would have impurity levels of 0.1% only. This is significantly less than the impurity levels found in AZACTAM® (commercially available aztreonam arginine), which contains 1.6% total impurities.

β-Aztreonam lysinate can be successfully manufactured from β-aztreonam according to the manufacturing process I, where the total impurities in the bulk solution are around 0.3% and around 0.45% in the lyophilizates. This represents an increase of around 0.1% versus API. However, when the manufacturing process II is used for preparation of β-aztreonam, the impurity level in the bulk solution is around 15.8%. After lyophilization, the impurity level remains unchanged. The analysis of the impurities profile show that the high impurity level in the β-aztreonam is mainly due to generation of the impurity B and a second unknown impurity, both generated during β-aztreonam lysinate salt conversion process. All other impurities typically found in the β-aztreonam bulk solutions and lyophilizates also remain lower than 0.1%.

These results indicate that the manufacturing process II is not a most suitable process for preparation of β-aztreonam lysinate. Not only it leads to the high increase in impurities but it also requires a dedicated manufacturing procedure and the costly equipment for production of the bulk solution. However, when the β-aztreonam lysinate is desired, the manufacturing process I is very suitable to prepare the product with acceptable degree of impurities.

As is seen from these results, α-aztreonam offers a major advantage for manufacturing α-aztreonam lysinate over β-aztreonam lysinate. α-Aztreonam can easily be suspended in water, forming a homogeneous suspension without need for any special equipment allowing a lysine solution to be added to the α-aztreonam.

Therefore, the bulk solutions and lyophilizates for α-aztreonam lysinate containing about 75 mg/mL can be manufactured in a straightforward, practical, fast, easy and inexpensive way.

### D. Advantages of α-Aztreonam Lysinate

Since the aztreonam containing arginine is not suitable for inhalation, other acid addition salts were prepared and tested. Aztreonam lysinate, particularly aztreonam lysinate derived from α-aztreonam form, was found to be pharmacologically most safe, acceptable and efficacious for inhalation purposes when administered by nebulization as a dry powder or aerosolized lyophilizates in amounts of about 75 mg/mL.

The prior art process dealing with α- and β-aztreonams involved conversion of α-aztreonam to β-aztreonam. In order to yield the aztreonam lysinate as a final product, such conversion step, if used for production of bulk or lyophilized aztreonam lysinate, necessarily involves reacting the β-aztreonam being relatively insoluble in water and having a pH of approximately pH 2.3, with the lysine salt having a pH of approximately pH 10. The addition of the lysine salt component to β-aztreonam creates excessive ion exchange during the titration of the aztreonam acid to a physiologically acceptable pH. Additionally, this reaction results in an undesirable side reaction with open chain formation of the β-lactam ring in aztreonam, further leading to β-aztreonam lysinate having a higher degree of impurity, instability and an undesirably high osmolality.

High osmolality is not desirable for the inhalable aztreonam. The inhalable aztreonam formulation requires very specific degree and range of osmolality because the high osmolality of the inhalable formulation may cause a patient to react to the inhalation with bronchospasm or cough.

In the current invention, the preferred pharmaceutically acceptable aztreonam lysinate salt is derived from a direct reaction of α-aztreonam with lysine without need of conversion of α-aztreonam to β-aztreonam first.

The production of α-aztreonam lysinate derived from α- aztreonam form without converting α-aztreonam into β-aztreonam is a novel process not disclosed or suggested by any prior art.

The currently disclosed process for manufacturing a bulk solution or lyophilized α-aztreonam lysinate for inhalation is based on the finding that α-aztreonam, when solubilized in water and stirred, immediately forms an emulsion or smooth slurry. When a lysine salt solution is titrated to the slurry, a rapid formation of an amorphous α-aztreonam lysine salt results. This salt has similar stability characteristics to the lyophilized β-aztreonam lysinate without, however, a detrimental increase in the impurities observed during a β-aztreonam lysinate production. The reaction with lysine, lyophilization and drying of the α-aztreonam lysinate does not cause the opening of the nucleophilic ring and thus the initial impurity levels generated from the alpha form is less than 0.1%, substantially less than the FDA limit for the permitted level of impurities.

Therefore, by using the α-aztreonam directly for formation of α-aztreonam lysinate, the obtained product contains much lower initial impurity levels, has a higher stability and over time shows lesser degradation thereby leading to the product with a longer shelf life.

In the current process for preparation of α-aztreonam lysinate from α-aztreonam, the basic salt conversion volumes, ratio of individual components and pH of the reaction mixture are titrated to a fixed level. The titration process confirms that less than 100 ppm of residual ethanol in the α-aztreonam lysinate remains using the manufacturing process II compared to the manufacturing of β-aztreonam lysinate where in the same reaction volume the residual ethanol levels up to 10,000 ppm were detected. These levels are approximately 100 times than those observed during α-aztreonam lysinate preparation. Moreover, by using the α-aztreonam, the formation of ethyl ester, another impurity detected in the β-aztreonam forms is eliminated.

Concerning the stability of the two formulations, the accelerated stability conditions shows that the β-aztreonam degrades from the initial 0.9% open chain to over 2% at 30 days whereas α-aztreonam degrades from an initial 0.06% to only 1.2% in 90 days under the same testing conditions.

Consequently, the use of the α-aztreonam and preparation of the α-aztreonam lysinate using the manufacturing process II produces a more stable product with a better pH profile, lower impurity content, longer stability and a desirably reduced osmolality.

### E. Process for Manufacturing of α-Aztreonam Lysinate

Three potential techniques were developed to yield the α-aztreonam lysinate derived from the α-aztreonam. The first technique involves titration of lysine salt into the α-aztreonam. The second techniques involves vacuum-drying of the raw α-aztreonam at the end point of the synthesis when the aztreonam is combined with lysine in a lyophilizer. In the third technique, α-aztreonam lysinate is produced directly. The third technique involves spray-drying of the α-aztreonam and lysine into a bulk solid producing the aztreonam lysinate as the final product. All these techniques avoid conversion of the α-aztreonam to the β-aztreonam.

The current preferred process for preparation of the aztreonam lysinate derived from α-aztreonam comprises solubilization of α-aztreonam in water and subsequent titration of an aqueous solution of lysine into the α-aztreonam to form the lysine salt. The mixture is then lyophilized or spray dried.

The current process avoids cleavage of the β-lactam ring by advantageously employing a titration to achieve a desirable pH profile of the α-aztreonam lysinate which is contrary to the techniques used for β-aztreonam lysine salt preparation. In either of the techniques disclosed herein for preparation of the α-aztreonam lysinate derived from the α-aztreonam, a conversion to the β-aztreonam as well as all problems connected with production of the β-aztreonam lysinate derived from the β-astreonam are avoided.

### e. α-Aztreonam Lysinate Manufacturing Process

Manufacturing process for preparation of a bulk solutions of α-aztreonam lysinate comprising about 75 mg of α-aztreonam per one ml of water or another aqueous solvent is essentially based on the previously described manufacturing process II. The process involves reaction of components listed below in Table 6.

**Table 6**

| Components | mg per 1 mL Unit |
|---|---|
| α-Aztreonam | 75 mg |
| Lysine monohydrate | 52.5 mg |
| Water for injection up to 1 mL | |
| Nitronen | qs |

For preparation of one liter of α-aztreonam lysinate, 75 grams of α-aztreonam and 52.5 grams of lysine monohydrate are dissolved in one liter of water for injection. For a large scale preparations of α-aztreonam lysinate, these amounts are appropriately multiplied.

Specifically, the process steps for preparation of the bulk solution are as follows. Approximately 400 mL of water for injection (WFI) is added to a mixing vessel, the mixing vessel is cooled to a temperature between 2 and 8°C and 52.5 grams of lysine monohydrate is added to the water. The temperature is maintained between 2 and 8°C. The mixture is then stirred until clear. The solution is brought to 500 mL volume with water for injection.

Separately, 400 mL of water for injection is added to the second mixing vessel and cooled to between to 2 to 8°C and 75 grams of anhydrous α-aztreonam is suspended in the cooled water under rapid stirring and cooling to keep the temperature under 10°C. The actual amount of α-aztreonam, which typically may contains up to 15% of moisture, is adjusted such as to correspond to 75 grams of anhydrous α-aztreonam. Then the calculated amount of the lysine solution is titrated over relatively short period of time between about 1 and 15 minutes, preferably in about 6 minutes, into the aztreonam suspension while maintaining the temperature constantly below 10°C and the pH equal to or less than 6.0. The pH of the solution is measured and adjusted, if needed, with lysine monohydrate solution to a final pH of 4.8 +/-0.5. The solution is brought to volume with water for injection and mixed until clear. The final volume of both the lysine solution and α-aztreonam solution, combined, should be one liter.

After verifying that the pH and assay results are within specification, the solution is filtered by means of peristaltic pump through a prefilter, preferably 0.45 µm prefilter, and through two additional filters, preferably of size of about 0.1 to about 0.3 µm, preferably a 0.22 µm hydrophilic Millidisk 40 cartridge filter, into the receiving vessel that is maintained under filtered (0.22 µm) nitrogen flow. The integrity of the final (0.22 µm) filter is tested after filtration. The receiving vessel is maintained at a temperature lower than 10°C. Post-filtration samples are tested for the amount of α-aztreonam lysinate, contamination, density and appearance.

The visual appearance of the α-aztreonam lysinate bulk solution was as a yellowish solution free of particulate matter. The pH of the bulk solution was 4.82. Viscosity, surface tension and osmolality were 1.55 mPas, 67.11 mN/m and 410 mOsmol/kg, respectively. Total impurities were 0.328%.

The filtered solution is filled into amber glass vials with a 1 mL ±10% fill volume. Fill volume are checked every 15 minutes. Vials are equipped with stoppers left in the open position and placed in the lyophilizer.

Lyophilization is performed according to three step exemplary lyophilization conditions summarized in Table 7.

**Table 7**

| Lyophilization Conditions | | |
|---|---|---|
| Step | Procedure | Lyophilization Process |
| 1 | Product freezing | Freezing conditions: |
| | | Shelves chilled: <-46°C |
| | | The product frozen at -10°C ±3°C with no vacuum and held for two hours |
| 2 | Primary drying | Drying conditions: |
| | | 1 hour: -40°C ±3°C and at ≤80 microbar |
| | | At least 15 hours : -25°C ± and ≤80 microbar |
| | | Temperature ramped to 25°C ±3°C with a gradient of 10°C/hour |
| 3 | Secondary drying | Drying conditions: |
| | | At least 10 hours: 25°C ±3°C and ≤80 microbar and until product reaches 25°C ±3°C |
| | | The vacuum broken with sterile nitrogen |

Alternatively, the lyophilization cycle can be performed under different conditions, such as for example, conditions listed in Table 8.

**Table 8**

| Lyophilization Conditions | | |
|---|---|---|
| Step | Procedure | Lyophilization Process |
| 1 | Product freezing | Freezing conditions: |
| | | Shelves chilled: -38°C |
| | | The product frozen at -35°C ±3°C with no vacuum and held for four hours |
| 2 | Primary drying | Drying conditions: |
| | | Vacuum started at ≤80 microbar |
| | | Temperature decreased to - 25°C ± 3°C and held for 8 hours at ≤80 microbar |
| 3 | Secondary drying | Drying conditions: |
| | | Vacuum adjusted to ≤47 microbar |
| | | Temperature ramped to +25°C ±3°C and held for 16 hours Cycle ended, vials closed, vacuum broken |

In the second cycle for lyophilization, the vials are closed within the lyophilizer under vacuum, at the end of the secondary drying.

It is to be understood that any variation in the lyophilization process is intended to be within the scope of this invention.

Throughout the whole manufacturing process, water content and visual inspection tests are performed. Following the lyophilization, the vials are completely closed with stoppers and fitted with aluminum crimp caps. Vials are visually checked for effective capping every 15 minutes during the capping and sealing process.

The lyophilized vials are kept and stored at the temperature between about -20° to 8°C which was found to be the optimal temperature for lyophilized product stability. Additionally, this temperature was found to be the most optimal temperature for maintaining the stability of the product during manufacturing.

Manufacturing process II used for manufacturing of α-aztreonam lysinate has been described above and the impurities profile is illustrated in Figure 4. The total impurities observed during a large scale manufacturing of α-aztreonam lysinate according to the process II are only slightly higher than impurities seen in API. Specifically, only traces (0.014%) of impurity B can be detected in α-aztreonam lysinate product and represent a 5 fold reduction in that particular impurity compared to the β-aztreonam bulk solutions and lyophilizates.

The visual appearance of the α-aztreonam lyophilizates was as a yellowish solution free of particulate matter. The pH of the lyophilizate was 4.78. Viscosity, surface tension and osmolality were 1.5 mPas, 70.34 mN/m and 430 mOsmol/kg, respectively. Total impurities were 0.471%. These results were reproducible in three independent runs of α-aztreonam lysinate manufacturing batches.

To confirm that the manufacturing process II represent the optimal conditions for manufacturing of α-aztreonam lysinate for inhalable purposes, investigations of the effect of the pH, temperature and bulk solution concentrations were performed. Further, the stability of the lyophilized product as well as the diluted product was also evaluated.

### b. Evaluation of Effect of pH and Temperature

Since the pH values played such an important role in distinction between α and β-aztreonam, pH and temperature effect was evaluated for optimization of the manufacturing process for manufacturing of α-aztreonam lysinate.

Briefly, α-aztreonam lysinate (75 mg/ml) at pH 4.8 at 20°C was cooled to 10°C and 2°C and the pH was measured every 5 seconds. There was no significant impact of the cooling to a temperature of 10 or 2°C on the pH of the α-aztreonam lysinate bulk solution. Consequently, the manufacturing process can conveniently be performed at temperatures between 2 and 20°C, however, as stated above, the process is optimally performed at 2-8°C.

Additionally, as already described above, α-aztreonam is readily dissolvable in water without polymerization, discoloration, solidifying and gelling, permitting a lysine solution to be added to the α-aztreonam solution without encountering high pH values during the process.

Aztreonam stability is pH dependent. Maximum stability of aztreonam is in a pH range between pH 4.2 and 7, with the optimal pH range being between 4.6 and 4.8. As described above, the pH of the α-aztreonam falls within the optimal pH range.

### c. Influence of Bulk Solutions on the Impurity Profile

Optimal concentration for amount of aztreonam in the aerosol is about 75 mg/ml, however, such concentration may differ for treatment of different conditions. To optimize the manufacturing process changes in concentration of aztreonam in the bulk solutions were investigated vis-a-vis limitations set by the freeze-drying process, the amount of water to be removed during the freeze drying and by the shape and size of the lyophilization vial where the product is stored.

Typically, low concentration lead to physically unstable product and high concentrations of the active ingredient compound in the bulk solution can be detrimental to the overall drying process. The shape and size of vials have an impact on the volume of the bulk solution and the concentration of the active ingredient therein.

When the bulk solutions of 25, 37.5 and 75 mg/ml of α-aztreonam lysinate were investigated for a level of impurities, the purest bulk solution was obtained at 37.5 mg/ml concentration. However, α-aztreonam at the 75 mg/ml concentration was found to have only slightly higher level of impurities and this level of impurities was at acceptable levels below or around 0.1% of impurities and, most importantly, the drug concentration met the required amount of the active compound needed for inhalation product.

### d. α-Aztreonam Stability

Alpha aztreonam, compared to β-aztreonam, permits production of aztreonam lysinate with a higher purity, better stability and longer shelf-life.

The α-aztreonam has advantages of better dispersion in water and lower residual ethanol (100 ppm) content than the β-aztreonam (10,000 ppm). This property results in lower total impurities during the salt conversion. Using the α- or β-aztreonam at the beginning of the manufacturing process results in the same comparable final drug product, an amorphous aztreonam salt, each however, having a different level of impurities and stability.

Two lots of drug product were produced using the β-aztreonam and one lot of drug product was produced using the α-aztreonam. The three lots of drug product were evaluated for stability at the time of the product manufacture (total initial impurities) and at 6 months after the manufacture (total impurities at 6 months) in the product stored under refrigeration at 5°C. The total impurity results for the lots are listed in the Table 8.

**Table 8**

| Total Impurities | | | |
|---|---|---|---|
| | Aztreonam form | Total initial impurities | Total impurities at 6 months |
| Drug product 1 | β-aztreonam lysinate | 1.23% | 1.47% |
| Drug product 2 | β-aztreonam lysinate | 1.27% | 1.35% |
| Drug product 3 | α-aztreonam lysinate | 0.65% | 0.84% |

The initial total impurities for the drug product (aztreonam lysinate) produced from β-aztreonam were 1.23% and 1.27%, respectively, as measured by HPLC, whereas the initial total impurities for the drug product produced from α-aztreonam were 0.65%. After 6 months of storage at 5°C, total impurities for β-aztreonam lysinate were 1.47% and 1.35%, respectively, whereas the total impurities for α-aztreonam lysinate were 0.84%.

Since the likely limit of impurities for the final product at a time of administration is 2%, the α-aztreonam lysinate is a better candidate for a stable product and thus a preferred aztreonam form leading to a product with a longer shelf life than the β-aztreonam lysinate, although β-aztreonam lysinate has also acceptable level of impurities.

### e. Stability of the Diluted α-Aztreonam

Since the inhalable α-aztreonam lysinate is, in one mode, delivered as an aerosolable solution, its stability in the solution was determined.

To that effect, α-aztreonam lysinate samples taken from the bulk solutions were diluted with a buffer at a ratio of 1:250. Samples were then investigated at a time zero (freshly prepared), after 1, 3, 6 and 12 hours and impurity profiles were determined.

The diluted samples of α-aztreonam lysinate were found to be stable for the entire 12 hours, with only negligible increases in impurities toward the 12 hour limit.

These finding show that there is a good stability of the α-aztreonam lysinate in a diluted state and that there is no rapid degradation of the α-aztreonam lysinate when the lyophilized product is diluted before its use as an inhalation product.

### a. Reconstitution of Lyophilized α-Aztreonam Lysinate

The impurity profile for reconstituted samples of lyophilized α-aztreonam lysinate was determined.

For this purpose, the lyophilizates were reconstituted with 1 ml of the 0.17% saline and visual appearance, pH, viscosity, surface tension, osmolality and total impurities were determined.

The visual appearance of the α-aztreonam lysinate lyophilizate was as a yellowish solution free of particulate matter. The lyophilizate's pH was 4.78. Viscosity, surface tension and osmolality were 1.5±0.16 mPas, 70.34±0.2 mN/m and
430 mOsmol/kg, respectively. Total impurities were 0.601%.

Osmolality of the inhalable product is extremely important. High osmolality is not desirable for the inhalable aztreonam as it is not tolerated by patients with respiratory infections. The inhalable aztreonam formulation requires very specific degree and range of osmolality because the high osmolality of the inhalable formulation may cause a patient to react to the inhalation with bronchospasm or cough.

It is therefore of great importance that both the lyophilized and reconstituted lyophilized α-aztreonam lysinate have acceptable osmolality in the range of around 400-550 mOsml/kg.

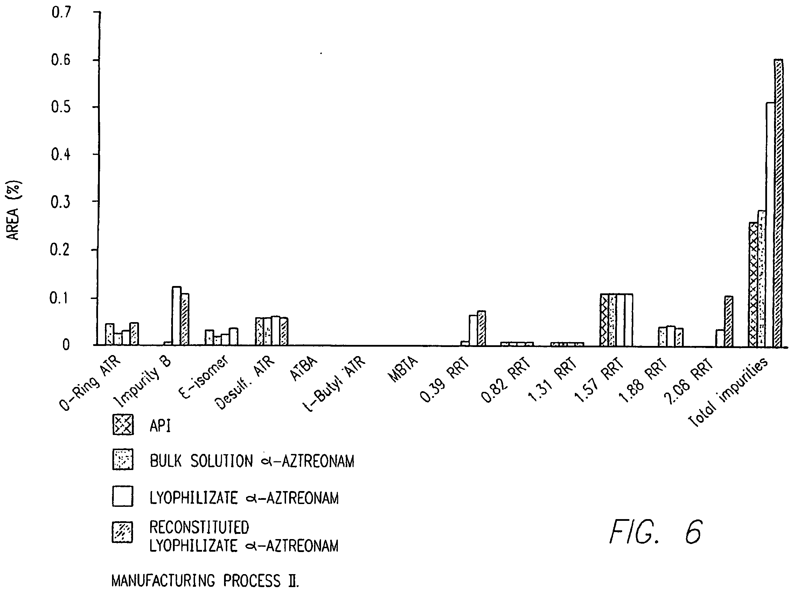

Figure 6 graphically illustrates the impurity profiles of the bulk solution, lyophilizate and reconstituted lyophilizate of α-aztreonam lysinate compared to API.

As seen from Figure 6, impurities profile of API, bulk solution and lyophilizate was comparable to the profiles seen in Figure 4. Reconstituted lyophilizates has shown overall slightly but insignificantly increased level of impurities in the reconstituted lyophilizates from about 0.471% to about 0.601%.

### F. Aztreonam Lysinate Pharmacological Activity

Aztreonam lysinate exhibits potent and specific activity in vitro against a wide spectrum of gram-negative aerobic pathogens including *Pseudomonas aeruginosa.* The bactericidal action of aztreonam lysinate results from the inhibition of bacterial cell wall synthesis due to a high affinity of aztreonam lysinate for penicillin binding protein 3 (PBP3).

Aztreonam lysinate, unlike the majority of β-lactam antibiotics, does not induce β-lactamase activity and its molecular structure confers a high degree of resistance to hydrolysis by β-lactamases, such as penicillinases and cephalosporinases, produced by most gram-negative and gram-positive pathogens. Aztreonam lysinate is therefore especially effective against gram-negative aerobic organisms that are resistant to antibiotics hydrolyzed by β-lactamases.

Aztreonam lysinate maintains its antimicrobial activity at a pH ranging from 6 to 8 *in vitro* as well as in the presence of human serum and under anaerobic conditions. Aztreonam lysinate is active *in vitro* and is effective in laboratory animal models and clinical infections against most strains of the following organisms, *Escherichia coli*, *Enterobacter* species, *Klebsiella pneumoniae*, *Klebsiella oxytoca*, *Proteus mirabilis*, *Pseudomonas aeruginosa*, *Serratia marcescens*, *Haemophilus influenzae*, and *Nitrobacter* species, including many that are multi-resistant to other antibiotics such as certain cephalosporins, penicillins, and aminoglycosides.

Currently, the only infections for which aztreonam arginine salt is FDA approved are those caused by *Escherichia coli*, *Klebsiella pneumoniae*, *Pseudomonas aeruginosa*, *Haemophilus influenzae*, *Proteus mirabilis*, *Enterobacter species* and *Serratia marcescens.*

It has now been discovered that all the above named bacterial strains as well as rare and highly resistant strains, such as *Burkholderia cepacia, Stenotrophomonas maltophilia, Alcaligenes xylosoxidans,* and multidrug resistant *Pseudomonas aeruginosa* are successfully eradicated by daily treatment with low doses between about 1 and about 250 mg, preferably about 75 mg/mL, of aztreonam lysinate, preferably administered once or twice a day, with total daily doses not exceeding 750 mg/day.

### II. Aztreonam Lysinate Inhalable Composition

A concentrated inhalable α- or β-aztreonam lysinate, preferably α-aztreonam lysinate, composition is described suitable for efficacious delivery of aztreonam lysinate into the endobronchial space of airways by aerosolization or as a dry powder.

It is most preferably suitable for formulation of concentrated aztreonam lysinate for aerosolization by atomizing, jet, ultrasonic, pressurized, vibrating porous plate or equivalent nebulizers or by dry powder inhalers which predominantly produce aztreonam lysinate aerosol or dry powder particles between 1 and 5 µ. Such particle sizes are necessary for efficacious delivery of aztreonam lysinate into the endobronchial space to treat bacterial infections.

### A. Aerosolized Aztreonam Lysinate Composition

Aztreonam lysinate composition for aerosolization is formulated for efficacious delivery of aerosolized aztreonam lysinate to the lung endobronchial space of airways.

The aerosol formulation is delivered in a total volume of between about 1 and about 5 ml of aqueous physiologically acceptable solution for one inhalation dose. When formulated and delivered as described, it delivers a therapeutically efficacious dose of aztreonam lysinate to the site of the infection in amount sufficient to treat bacterial pulmonary infections.

A combination of the novel aqueous formulation with the atomizing, jet, pressurized, vibrating porous plate or ultrasonic nebulizer permits, depending on the nebulizer, about at least 20 to about 90%, typically about 70% delivery of the administered dose of aztreonam lysinate into airways.

The formulation contains a minimal yet efficacious amount of aztreonam lysinate from about 1 to about 250 mg, more preferably from about 25 to about 90 mg/mL, and most preferably about 75 mg/mL, formulated in the smallest possible volume of physiologically acceptable diluent having a certain degree of salinity and certain pH, adjusted to permit generation of an aztreonam lysinate aerosol well tolerated by patients but minimizing the development of secondary undesirable side effects such as bronchospasm, inflammation or cough.

Primary requirements for aerosolized aztreonam lysinate formulation are its safety and efficacy. Additional advantages are lower cost, manufacturing convenience, purity of the product, practicality of use, long shelf-life, storage and manipulation of the aerosol device. These requirements for aerosolized aztreonam lysinate have now been found to be met by the formulation containing certain degree of salinity and have certain pH range.

### 1. Dosage of Aztreonam Lysinate

Aztreonam lysinate has a relatively short life-time. Its half life time is about 1-2 hours and within ten to twelve hours the whole aztreonam dose is eliminated. Consequently, the effective treatment of bacterial pulmonary infections requires a treatment regimen which provides sufficient amount of drug to maintain the antibacterial level of aztreonam lysinate in the lung. Such regimen thus requires administration of an inhalable aztreonam lysinate one to several, preferably two to four, times a day. Most preferred dosing regimen for patient convenience is once or twice a day, however, because of a specific effect aztreonam lysinate asserts on the bacteria, and because of its relatively short life-time of about 12 hours, more than twice a day dosing is often required for complete eradication of the bacteria from the endobronchial space.

It is therefore preferable to deliver aerosolized or dry powder aztreonam lysinate in a smallest therapeutically efficacious amount at least twice a day, in some instances three to four times, and exceptionally more than four times a day. A dose of aztreonam lysinate is therefor set to be between 1 and 250 mg per one dose formulated in, most preferably, about 75 mg of aztreonam/mL.

Typically, one therapeutically effective dose contains between 1 and 250 mg, preferably between 25 to 90 mg of aztreonam lysinate, in equivalent, administered by means that provides at least about 50%-70% efficacy of aztreonam lysinate delivery to the endobronchial space. Thus, with about a 250 mg dose, 125 mg of aztreonam lysinate is delivered during each administration. 100-250 mg of aztreonam lysinate delivered to the lung has been found to be efficacious in eradication of bacteria. In no instance should one dose exceed 250 mg. Above this amount, aerosolization is difficult, the drug tends to precipitate, and larger volumes are necessary for its delivery by aerosol, which defeats the purpose of the invention to deliver the therapeutical amount of drug with the greatest efficiency.

Determination of effective dosage of administered aztreonam lysinate and the regimen used for treatment of each patient depends on the responsiveness of the individual patient to the treatment. The ultimate decisive factor is the expected level of aztreonam lysinate in the sputum after aerosolization. The optimal range of aztreonam lysinate in 1 mL of sputum at any given time should be in the 500 to 2000 µg/mL range. Thus, the frequency of the administration is correlated with the effectiveness of administered aztreonam lysinate.

The effectiveness of aerosolized aztreonam lysinate is surprisingly high when compared to effectiveness of the intravenously administered aztreonam lysinate where the serum peak levels following the maximum permitted dose 2,000 mg resulted only in 242 µg/mL of sputum. Following such intravenous administration, the 6 hours levels were found to be in the range of 16 µg/mL, which is the MIC for non-resistant *Pseudomonas aeruginosa.*

The new mode of administration permitting a noninvasive
administration of small yet effective amounts of aztreonam lysinate directly into lungs is a great improvement compared to all previously known method used for delivery of aztreonam lysinate.

### 2. Effect of pH on Aztreonam Lysinate Formulation

The solution or diluent used for preparation of aztreonam lysinate aerosol has a limited pH range from 4.2 to 7.5, preferably between 5.5 and 7.0.

The pH of the formulation is an important feature for aerosolized aztreonam lysinate delivery. When the aerosol is either acidic or basic, it can cause bronchospasm and cough. Although the safe range of pH is relative and some patients may tolerate a mildly acidic aerosol, others, particularly those with cystic fibrosis or other underlying disease will experience bronchospasm. Any aerosol with a pH of less than 4.5 typically induces bronchospasm. Aerosols with a pH between 4.5 and 5.5 will cause bronchospasm occasionally. Testing with aztreonam lysinate aerosol discovered that an aerosolizable aztreonam lysinate formulation having a pH between 5.5 and 7.0 is well tolerated and safe. Any aerosol having pH greater than 7.5 is to be avoided as the body tissues are unable to buffer alkaline aerosols. Aerosol with controlled pH below 4.5 and over 7.5 causes lung irritation accompanied by severe bronchospasm, cough and inflammatory reactions.

For these reasons as well as for the avoidance of bronchospasm, cough or inflammation in patients, the optimum pH for the aerosol formulation was determined to be between pH 5.5 to pH 7.0.

Consequently, the aztreonam lysinate aerosol formulation is adjusted to pH between 4.5 and 7.5 with preferred pH range from about 5.5 to 7.0. Most preferred pH range is from 5.5 to 6.5.

### 3. Effect of Salinity on the Aztreonam Lysinate Formulation

Patients suffering from acute or chronic endobronchial infections and particularly those with cystic fibrosis or bronchiectasis have increased sensitivity to various chemical agents and have high incidence of bronchospastic, asthmatic or cough incidents. Their airways are particularly sensitive to hypotonic or hypertonic and acidic or alkaline conditions and to the presence of any permanent ion, such as chloride. Any imbalance in these conditions or the absence of chloride below certain values leads to bronchospastic or inflammatory events and/or cough which greatly impair treatment with inhalable formulations. Both these conditions prevent efficient delivery of aerosolized aztreonam lysinate into the endobronchial space. The clinical manifestations of the irritated airways are extremely undesirable.

Clearly, for aztreonam lysinate, it is not possible to use solely an aqueous solvent without providing certain degree of osmolality to meet and emulate physiological conditions found in healthy lungs. Consequently, certain amount of the chloride or another anion is needed for successful and efficacious delivery of aerosolized aztreonam lysinate but such amount is much lower than amounts provided and typically used for aerosols of other compounds.

Bronchospasm or cough reflexes do not respond to the same osmolality of the diluent for aerosolization, however, they can be sufficiently controlled and/or suppressed when the osmolality of the diluent is in a certain range. Preferred solution for nebulization of aztreonam lysinate which is safe and has airways tolerance has a total osmolality between 50 and 550 mOsm/kg with a range of chloride concentration of between 31 mM and 300 mM. The given osmolality controls bronchospasm, the chloride concentration, as a permeant anion, controls cough. In this regard the chloride anion can be substituted with bromine or iodine anions, since both are permeant anions. In addition, bicarbonate may be wholly or partially substituted for chloride ion. Normal saline (NS) contains 154 mM of chloride whereas 31 mM of chloride corresponds to about 0.2 normal saline.

Consequently, the formulation for aztreonam lysinate aerosol comprises from about 1 to about 90 mg, preferably about 75 mg, of aztreonam lysinate dissolved in 1 mL of a normal, or preferably a diluted saline to from about 1/10 normal saline (NS) to about and at most to 1 NS solution, preferably from about 1/10 to about 1/4 NS, that is a one tenth to one quarter diluted normal saline. It has now been discovered that aztreonam lysinate is efficaciously delivered into lungs when dissolved in lesser than normal saline, that is saline containing 0.9% of sodium chloride, and that the concentration of a chloride ion equal to or lesser than 1/4 N saline permits and assures a delivery of aztreonam lysinate into endobronchial space.

The aztreonam lysinate formulation containing about 50 mg of aztreonam lysinate per 1 mL of 0.2 NS has an osmolality of about 290 mOsm/L. Such osmolality is within a safe range of aerosols suitable for administration to patients suffering from pulmonary bacterial infections and also those patients with a cystic fibrosis or bronchiectasis.

An additional feature and advantage of using 1/10 to 1/4 NS solution comprising 50 mg/mL aztreonam lysinate is that the resulting aerosol formulation is very efficiently nebulized by an atomic, jet or ultrasonic nebulizer compared to aztreonam lysinate dissolved in a normal saline. Since the delivery of aztreonam lysinate formulated as described herein is much more efficient, much lower amount of aztreonam lysinate is needed to achieve complete eradication of gram-negative bacteria in lungs. Instead of 1000 to 4000 mg of aztreonam which was shown to be somehow effective in the only one prior attempt to aerosolize aztreonam, the formulation of aztreonam lysinate described herein permits treatments with as little as 1 mg/mL and with at most up to 50 mg/mL of aztreonam lysinate in a maximum amount of 5 mL volume, delivered preferably with an atomizing, jet, electronic or ultrasonic nebulizer.

### 5. Dry Powder, Aerosol and Aerosol Suspensions

The formulation according to the invention contains aztreonam lysinate formulated as a dry powder, aerosol solution or aerosol suspension of liposomes or other microscopic particles in an aqueous solvent. The formulation is designed to be well tolerated and able to be reliably and completely nebulized to aerosol particles within the respirable size range of 1 to 5 µ.

The doses are designed to contain as much as, but not more than, the necessary amount of a most active form of aztreonam lysinate to prevent colonization and/or to treat severe pulmonary infections caused by a range of susceptible gram-negative organisms.

Patients can be sensitive to pH, osmolality, and ionic content of a nebulized solution. Therefore these parameters are adjusted to be compatible with aztreonam lysinate chemistry and still tolerable to patients.

The formulation is nebulized predominantly into particle sizes allowing a delivery of the drug into the terminal and respiratory bronchioles where the bacteria reside during infection and in the larger airways during colonization.

For efficacious delivery of aztreonam lysinate to the lung endobronchial space of airways in an aerosol particle, the formation of an aerosol having a MMAD predominantly between 1 to 5 µ is necessary. The formulated and delivered amount of aztreonam lysinate for treatment and prophylaxis of endobronchial bacterial infections must effectively target the lung surface. The formulation must have a smallest possible aerosolizable volume able to deliver an effective dose of aztreonam lysinate to the site of the infection. The formulation must additionally provide conditions which would not adversely affect the functionality of the airways. Consequently, the formulation must contain enough of the drug formulated under the conditions which allow its efficacious delivery while avoiding undesirable reactions. The new formulation meets all these requirements.

One way to deliver inhalable aztreonam lysinate is by way of dry inhalable powder.

The aztreonam lysinate prepared according to the invention may be endobronchially administered in a dry powder formulation for efficacious delivery of the finely milled aztreonam powder into the endobronchial space using dry powder or metered dose inhalers as an alternative to aerosol delivery.

A dry powder formulation has potency, on a mass basis, which allows such alternative delivery of aztreonam lysinate as a dry powder using dry powder inhaler. A sufficiently potent formulation of aztreonam lysinate provides a dry powder which can be advantageously delivered by dry powder inhaler or by metered dose inhaler. For delivery of dry inhalable powder, aztreonam lysinate is milled, precipitated, spray dried or otherwise processed to particle sizes between about 1 and 5 µ.

Dry powder formulation comprises from about 20 to 200 mg, preferably 10 to 100 mg of aztreonam lysinate.

For dry powder formulation prepared according to the invention, aztreonam lysinate is milled to a powder having mass median average diameters ranging from 1 - 5 microns by media milling, jet milling, spray drying or particle precipitation techniques as described in Example 6.

Briefly, for spray drying, aztreonam alpha form is suspended in water, stirred and cooled. L-Lysine dissolved in water is added slowly over about 3 to about 10 minutes, preferably about 6 minutes, until both components are almost completely dissolved. Solution is purified using a charcoal and filtered. Subsequently, the solution is spray dried using any suitable spay-drying equipment, such as, for example Buchi Mini Spray Dryer B-191.

Particle size determinations are made using a multistage Anderson cascade impactor or other suitable method. The Thermo Andersen Eight Stage Non-Viable Cascade Impactor is specifically cited within the US Pharmacopoeia Chapter 601 as a characterizing device for aerosols within metered-dose and dry powder inhalers. The Eight Stage Cascade Impactor utilizes eight jet stages enabling classification of aerosols from 9.0 micrometers to 0.4 micrometers (at 28.3 L/min) and allows airborne particulate to impact upon stainless steel impaction surfaces or a variety of filtration media substrates. A final filter collects all particles smaller than 0.4.

Media milling is accomplished by placing a drug substance into a mill containing, for example, stainless steel or ceramic balls and rotating or tumbling the material until the desired drug particle size ranges are achieved. Advantages of media milling include good size control, narrow product size ranges, high efficiencies of recovery, and readily scalable processes. Disadvantages include long manufacturing process times which takes from several hours to several days, the requirement that the milling media be separated from the product at completion, and the possibility of contamination of the product with the media.

Jet milling uses very high pressure air streams to collide particles with one another, with fine particles of the desired size being recovered from the mill. Advantages include rapidity of the manufacturing process and less energy transfer during milling, resulting in less temperature rise during the drug production. The jet milling process is completed in seconds to minutes. Disadvantages of the jet milling include poorer yield and collection efficiencies, with only 50 to 80% of recovery being a typical yield.

Spray-drying is another technique useful for preparation of inhalable dry powder. Spray drying involves spraying a fine mist of aztreonam lysinate solution onto a support and drying the particles. The particles are then collected. Spray drying has the advantage of being the least prone to degrading chemical entities. Adding a co-solvent which decreases the solubility of a drug to a uniform drug solution results in solution precipitation. When sufficient co-solvent is added, the solubility of the drug falls to the point where solid drug particles are formed which can be collected by filtration or centrifugation. Precipitation has the advantage of being highly reproducible, having a high yield of recovery and being able to be performed under low temperature conditions, which reduce degradation.

Dry powder inhalation and metered dose inhalations are more practical when administered doses result in the delivery of at least about 10 mg, and more preferably about 25 to about 100 mg, of aztreonam lysinate to the lung of the patient receiving treatment. Depending on the efficiency of the dry powder delivery device, which is typically about 70%, typical effective dry powder dosage levels fall in the range of about 20 to about 60 mg of aztreonam lysinate. Therefore, typically more than one breath of drug is required.

In this aspect, a sufficiently potent formulation is provided of pure aztreonam lysinate in dry powder or metered dose form of drug particles milled or otherwise prepared to particle sizes predominantly with a range of 1 to 5 microns. Such formulation is practical and convenient because it does not require any further handling such as diluting the dry powder or filling an aerosol container. Further, it utilizes the devices that are sufficiently small, fully portable and do not require, for example, an air compressor which is needed for a jet nebulizer. Additionally, the dry powder formulation has a longer shelf life that the liquid aztreonam lysinate formulations for aerosolization. Aztreonam lysinate, when reconstituted into an aerosolizable solution, has only a limited shelf life at room temperature due to hydrolysis of the monobactam ring. Aztreonam lysinate dry powder does not have this problem.

The dry powder formulation is thus practical and convenient for ambulatory use because it does not require dilution or other handling, it has an extended shelf-life and storage stability and the dry powder inhalation delivery devices are portable and do not require an air compressor needed by aerosol nebulizers.

All techniques suitable for preparation of dry inhalable powders and any and all improvements thereof as well as any dry powder inhaler are intended to be within the scope of the invention.

### B. Stability, Shelf-Life and Storage

Stability of the formulation is another very important issue for efficacious formulation. If the drug is degraded before aerosolization, a smaller amount of the drug is delivered to the lung thus impairing the treatment efficacy. Moreover, degradation of stored aztreonam lysinate may generate materials that are poorly tolerated by patients.

The dry form of aztreonam lysinate has at least 2 years long shelf life. A long-term stability of aztreonam free base or aztreonam lysinate in aqueous solutions may not provide a sufficiently long shelf life which would be commercially acceptable. A liquid formulation, therefore, may require a separation of aztreonam lysinate from the appropriate diluent. For this reason, the formulation is preferably supplied in a dry form and can be a reconstituted prior to administration as described below.

A formulation for aerosolization is thus preferably provided as two separate components, one containing a dry aztreonam lysinate containing an appropriate diluent such as 0.1 to 0.9 N saline, bicarbonate, water for injection or any equivalent acqueous solution, as described above. The formulation is reconstituted immediately prior to administration. This arrangement prevents problems connected with the long-term stability of aztreonam lysinate in aqueous solvents.

According to the invention, aztreonam lysinate for aerosolization is preferably formulated in a lyophilized dosage form intended for use as a dry powder for reconstitution before inhalation therapy. The formulation of aztreonam lysinate can be aseptically prepared as a lyophilized powder either for dry powder delivery or for reconstitution and delivery, or as a frozen solution, a liposomal suspension, or as microscopic particles. The storage suitability of the formulation allows reliable reconstitution of the formulated aztreonam lysinate suitable for aerosolization.

### c. Formulation for Inhalation-Packaging

The formulation of the invention is packaged for delivery to a patient in a package comprising several components.

Exemplary formulation package, consists of two separately packaged components: the lyophilized aztreonam lysinate powder and the sterile saline diluent to reconstitute the powder prior to delivery by nebulization.

Each vial contains 90-110% of labeled amount of aztreonam (75 mg) and lysine (47 mg) as aztreonam lysinate. Aztreonam and lysine form an ionic salt, which readily dissolves in saline. The diluent is a sterile 1 mL vial of 0.17% sodium chloride inhalation solution (0.17mg/mL NaCl). After reconstitution with 0.17% NaCl, the pH of the solution is 4.2 - 7.0 and the osmolality is from 350 to 500 mOsmol/kg. The aztreonam related impurities are the following: open-chain aztreonam, desulfonated aztreonam, aztreonam E-isomer, and t-butyl-aztreonam. The total impurities are less than 1%. Each known contaminant is less than < 0.2%. Unknown impurities are less than <0.1%. All ingredients meet USP requirements with the exception of lysine monohydrate, which currently has no monograph in the USP. The formulation contains no preservatives.

### c. Administration of Aztreonam Lysinate by Inhalation

Aztreonam lysinate is currently not available.

### A. Two Modes of Inhalable Administration

Administration of inhalable aztreonam lysinate is achieved either with aztreonam lysinate aerosol or with inhalable dry aztreonam lysinate powder.

An arginine free formulation delivered by inhalation has been shown to safely treat respiratory infections caused by all susceptible gram-negative bacteria including *Pseudomonas aeruginosa, Escherichia coli, Klebsiella pneumoniae, Pseudomonas* aeruginosa, *Haemophilus influenzae*, *Proteus mirabilis*, *Enterobacter* species and *Serratia marcescens,* as well as, and more importantly, antibiotics resistant, strains *Burkholderia cepacia*, *Stenotrophomonas maltophilia*, *Alcaligenes xylosoxidans*, and multidrug resistant *Pseudomonas aeruginosa.*

### B. Frequency of Dosing

Treatment of pulmonary infections caused by the above named bacteria is achieved by a treatment regimen which provides one to several, preferably one to two, times a day an inhalable aztreonam lysinate. Most preferred dosing regimen for patient covenience is once or twice a day, however, because of a specific effect aztreonam lysinate asserts on the bacteria, and because of its relatively short life-time of about 12 hours, more often dosing is sometimes required for complete eradication of the bacteria from the endobronchial space.

In patients with severely impaired lung function, the frequency of dosing may be increased up to about twelve times a day each time, providing only such amount of aztreonam lysinate as necessary to maintain therapeutic level in the lung.

Aztreonam lysinate kills bacteria by lysing cell walls as long as the local concentration of antibiotic exceeds the bacteria minimal inhibitory concentration (Med. Clinics N. Am., 79: 4, 733-743 (1995)). Because of the relatively rapid clearance of antibiotics from the respiratory tract due to mucociliary action, greater efficacy is obtained at a lower dose of administered aztreonam lysinate by treating a patient three, four or more times a day rather than administer the drug only once or twice. To this effect the aztreonam lysinate dose delivered by inhalation is at least four times and can be one thousand time lower then the aztreonam arginine dose delivered intravenously or utilized in the one attempt described above to deliver aztreonam arginine by aerosolization where 500-1000 mg was delivered twice a day to a total amount of 1000 mg for children under 5 years of age and 2000 mg for individuals older than 5 years.

The current daily dose of aztreonam lysinate can be as small as 2 mg. The typical upper limit is 500 mg of aztreonam lysinate per day delivered in two to four administrations. In extreme cases the dose may reach up to 750 mg per day delivered in three, four or more aerosol administrations. Typical and preferred range for one aerosol dosage is between 20 and 200 mg administered twice a day or between 10 and 100 mg administered three or four times per day. Most preferred dose is 75 mg/mL delivered twice or more times a day.

Aerosolization of aztreonam lysinate utilizes delivery of aerosolized aztreonam lysinate using atomizing, jet, ultrasonic, electronic or other equivalent nebulizers. Portable nebulizers, such as atomizing, ultrasonic and electronic nebulizers are preferred for ambulatory treatment. The jet nebulizers with a compressor nebulize the aztreonam lysinate formulation very efficiently but are more suitable for use in the hospital and doctor's office.

A dry powder inhalation, as the second mode of administration of the inhalable aztreonam lysinate utilizes the aztreonam lysinate dry powder formulation. Such formulation comprises a delivery of the finely milled azreonam lysinate directly to the endobronchial space. In this instance, aztreonam lysinate is delivered into the endobronchial space using dry powder or metered dose inhalers. The aztreonam lysinates potency, determined on a mass basis, allows the inhalation of aztreonam lysinate powder, as an alternative mode of administration to the aerosol. Dry powder inhalation is most efficacious, practical and economical when administered doses contain less than 100 mg. The frequency of dosing, thus, is typically three or four times a day but also includes one or two or more than four times dosing regimen as this regimen depends on the need and condition of the patient.

A sufficiently potent formulation of aztreonam lysinate is described in a form of dry powder delivered as metered dose inhalation of aztreonam lysinate particles milled or spray dried to particle sizes predominantly within a range of 1 to 5 µ. Such dry powder delivery is possible and preferable particularly for ambulatory inhalation as it simplifies the delivery process. Such delivery is convenient because it does not require any further handling such as diluting the dry powder or mixing the powder with a solvent, etc. Further, the dry powder inhalation utilizes the devices that are sufficiently small, fully portable and do not require, for example, an air compressor which is needed for a jet nebulizer. Additionally, the dry powder formulation has even longer shelf life than the liquid aztreonam lysinate formulation for aerosolization.

The dosing regimen for both aerosol and dry powder aztreonam lysinate comprises from one to four, typically, or more than four times daily, in untypical cases, administration of the aerosol or dry powder.

Severely impaired cystic fibrosis patients, for example, may be able to withstand only one inhalation at a time but could repeat this inhalation of small amount of aztreonam lysinate every two, three or four hours to obtain sufficient level of aztreonam lysinate in the lungs.

### IV. Devices for Delivery of Aerosolized Aztreonam Lysinate

A primary requirement is to deliver aztreonam lysinate efficiently to the endobronchial space of airways in a most economic way. Thus, at least 30-50%, preferably 70-90% of the active drug, that is aztreonam lysinate subjected to nebulization is in fact delivered to a site where it asserts its therapeutic effect.

### A. Nebulizers

The composition described above provides the drug formulated in a solution permitting delivery of a therapeutically efficient amount of the drug, provided that the aerosol generated by the nebulization meets criteria required for such efficient delivery. The apparatus (nebulizer) which aerosolizes the formulation of aztreonam lysinates thus becomes a very important part.

There are quite a few nebulizer types currently commercially available. Not all of them are suitable for practicing the method described here.

A nebulizer is selected primarily on the basis of allowing the formation of aztreonam lysinate aerosol having a MMAD predominantly between 1 to 5 µ. The delivered amount of aztreonam lysinate must be efficacious for treatment and prophylaxis of endobronchial infections, particularly those caused by susceptible bacteria. The selected nebulizer thus must be able to efficiently aerosolize the formulation which has salinity, osmotic strength, and pH adjusted as to permit generation of aztreonam lysinate aerosol that is therapeutically effective and well tolerated by patients. The negulizer must be able to handle the formulation having a smallest possible aerosolizable volume and still able to deliver effective dose of aztreonam lysinate to the site of the infection. Additionally, the aerosolized formulation must not impair the functionality of the airways and must minimize undesirable side effects.

The inability of certain nebulizers to nebulize therapeutic quantities of drugs into small and uniform particle size aerosols is well known. For efficacious delivery of aztreonam lysinate a range of aerosolized particles with MMAD needed to deliver the drug to the endobronchial space, the site of the infection, is between 1-5 µ. Many commercially available nebulizers are able to aerosolize large volumes of the solution with an aim to deliver at least 10% of the volume to the endobronchial space by producing around 90% of large aerosol particles above 5 µ with a very large number of particles being in the range of 50-100 µ. These nebulizers are inefficient and not suitable for delivery of aztreonam lysinate.

In order to be therapeutically effective, the majority of aerosolized aztreonam lysinate particles should not have larger than between 1 and 5 µ. When the aerosol contains a large number of particles with a MMAD larger than 5 µ, these are deposited in the upper airways decreasing the amount of antibiotic delivered to the site of infection in the lower respiratory tract.

Previously, two types of nebulizers, jet and ultrasonic, have been shown to be able to produce and deliver aerosol particles having sizes between 1 and 5 µ. These particle size are optimal for treatment of pulmonary bacterial infection cause by gram-negative bacteria such as *Pseudomonas aeruginosa, Escherichia coli, Enterobacter* species, *Klebsiella pneumoniae, K. oxytoca, Proteus mirabilis, Pseudomonas aeruginosa, Serratia marcescens, Haemophilus influenza, Burkholderia cepacia, Stenotrophomonas maltophilia, Alcaligenes xylosoxidans,* and multidrug resistant *Pseudomonas aeruginosa*. However, unless a specially formulated solution is used, these nebulizers typically need larger volumes to administer sufficient amount of drug to obtain a therapeutic effect. Therefore, without a specially formulated aztreonam lysinate the efficient delivery of aztreonam lysinate is not achieved.

Suitable nebulizers must be able to nebulize a small volume of the formulation efficiently, that is into the aerosol particle size predominantly in the range from 1-5 µ. Predominantly in this application means that at least 70% but preferably more than 90% of all generated aerosol particles are within 1-5 µ range.

Jet and ultrasonic nebulizers can produce and deliver particles between the 1 and 5 µ particle size. A jet nebulizer utilizes air pressure breakage of an aqueous aztreonam lysinate solution into aerosol droplets. An ultrasonic nebulizer utilizes shearing of the aqueous aztreonam lysinate solution by a piezoelectric crystal.

Typically, however, the jet nebulizers are only about 10% efficient under clinical conditions, while the ultrasonic nebulizer are only about 5% efficient. The amount deposited and absorbed in the lungs is thus a fraction of the 10% in spite of the large amounts of the drug placed in the nebulizer.

One type of nebulizer which is suitable and preferred for aztreonam lysinate delivery is an atomizing nebulizer which consists of a liquid storage container in fluid contact with the diaphragm and inhalation and exhalation valves. For administration of the aztreonam lysinate formulation, 1 to 5 mL of the formulation is placed in the storage container, aerosol generator is engaged which produces atomized aerosol of particle sizes selectively between 1 and 5 µ.

Typical nebulizing devices suitable for practicing the method include atomizing nebulizers, or modified jet nebulizers, ultrasonic nebulizers, electronic nebulizers, vibrating porous plate nebulizers, and energized dry powder inhalers modified for handling small volume of highly concentrated drug in a specific formulation having a specific pH, osmolality and salinity. Most preferred nebulizer is the PARI inhalation nebulizer described in PCT/US00/29541 modified to meet the requirements of the method.

### B. Dry Powder Inhalers

Dry powder is administered as such using devices which deliver the dry powder directly to the lungs.

There are two major designs of dry powder inhalers. One design is the metering device in which a reservoir for the drug is placed within the device and the patient adds a dose of the drug into the inhalation chamber. The second is a factory-metered device in which each individual dose has been manufactured in a separate container. Both systems depend upon the formulation of drug into small particles of mass median diameters from 1 to 5 microns, and usually involve co-formulation with larger excipient particles (typically 100 micron diameter lactose particles). Drug powder is placed into the inhalation chamber (either by device metering or by breakage of a factory-metered dosage) and the inspiratory flow of the patient accelerates the powder out of the device and into the oral cavity. Non-laminar flow characteristics of the powder path cause the excipient-drug aggregates to decompose, and the mass of the large excipient particles causes their impaction at the back of the throat, while the smaller drug particles are deposited deep in the lungs.

Current technology for dry powder inhalers is such that payload limits are around 100 mg of powder. The lack of long-term stability of aztreonam lysinate in an aqueous solution due to hydrolysis allows dry powder inhaler technology to become a preferred delivery vehicle for aztreonam lysinate dry powder.

### C. Aerosol or Dry Powder Particle Size

Particle size of the aztreonam lysinate aerosol formulation is one of the most important aspects of the invention. If the particle size is larger than 5 µ then the particles are deposited in upper airways. If the particle size of the aerosol is smaller the 1 µ then it does not get deposited in the endobronchial space but continues to be delivered into the alveoli and may get transferred into the systemic blood circulation.

A jet nebulizer utilizes air pressure to break a liquid solution into aerosol droplets. An ultrasonic nebulizer works by a piezoelectric crystal that shears a liquid into small aerosol droplets. A pressurized nebulization system forces solution under pressure through small pores to generate aerosol droplets. A vibrating porous plate device utilizes rapid vibration to shear a stream of liquid into appropriate droplet sizes. However, only some formulations of aztreonam lysinate can be efficiently nebulized as the devices are sensitive to pH and salinity.

In dry powder inhalers, the aztreonam lysinate dry powder prepared as described above in dosages from 1-100 mg, preferably from 10-50 mg of dry powder as particles having sizes between 1 and 5 µ, is used directly.

### D. Efficacy of Aztreonam Lysinate Nebulization

Selection and choice of the nebulizer greatly effects efficacy of the inhalable aztreonam lysinate delivery.

A combination of an aerosol formulation of aztreonam lysinate and a nebulizing device significantly enhance the efficiency and speed of drug administration. Currently, for example the average time for administration of other aerosolized drugs, such as for example tobramycin, is 15-20 minutes per dose. The time required for this treatment represents a significant burden to the patient and contribute to reduced compliance with the BID regimen.

Furthermore, the nebulizer system used for tobramycin administration is less efficient than new atomizing devices. The total deposited dose of tobramycin in the lung is in the 12 to 15% range. Approximately 30% of the dispensed drug remains in the nebulizer at the end of treatment, and of the portion that is aerosolized, about 30% is emitted as particles too large or small to reach the lower airways.

The novel atomizing nebulizer, with an output of 8 to 10 microliters/seconds, or 0.48 to 0.60 mL/minute, is capable of delivering drug material 2 to 4 times faster than the prior nebulizers exemplarized by PARI LC plus nebulizer. Furthermore, the novel nebulizer is able to aerosolize approximately 90% of the dispensed dose, with 85% or more of the aerosol particles being within the size range required for lower airway deposition. As a result, administration of a specifically designed formulation of aztreonam lysinate using the atomizing nebulizer leads to substantial improvement in local delivery to the airways, to a shorter time required for delivery and, depending on the final concentration of aztreonam lysinate solution, reduces treatment time to as little as three or four minutes.

### V. Supporting Experimental Studies

*Pseudomonas aeruginosa* is the most common cause of chronic endobronchial infection in cystic fibrosis (CF) patients. This infection is a major cause of morbidity and mortality in these patients. Topical application of antibiotic agents inhaled as aerosol mists has demonstrated significant benefit to CF patients. Aerosolized antibiotic therapy with agents including carbenicillin, gentamicin, ticarcillin, tobramycin, and colistin but not aztreonam has been practiced for many years.

The most widely used aerosolized antibiotic for treatment of CF patients is tobramycin, which produces substantial improvements in pulmonary function and other clinical parameters. *In vitro*, tobramycin is active against most *P. aeruginosa* organisms in the absence of sputum; however, in the presence of sputum, tobramycin bioactivity is significantly reduced.

Aztreonam is a monobactam antibiotic with excellent activity against many aerobic gram-negative bacteria, including *P. aeruginosa.* It is currently approved as parenteral therapy for a variety of serious infections and has been widely used in control of pulmonary exacerbations in CF patients. Aztreonam has an antibacterial spectrum similar to the aminoglycoside antibiotics tobramycin and gentamicin. Its excellent activity against many aerobic gram-negative bacteria, including *P. aeruginosa,* has led to widespread use among CF patients, including intravenous administration as single agent therapy and in combination with other antibiotics for treatment of pulmonary exacerbations. These studies have demonstrated improvement in pulmonary function and clinical scores, as well as reductions in bacterial load and white blood cell counts. Additionally, aztreonam have been shown to have a potential for control of *Burkholderia cepacia*, a pathogen intrinsically resistant to the commonly used aminoglycoside antibiotics.

In order to determine whether aztreonam would be successful for treatment of *P. aeruginosa* and other bacterial infections, in the presence of sputum or mucin antagonized aztreonam bioactivity in vitro was investigated.

Experimental conditions are described in Example 8.

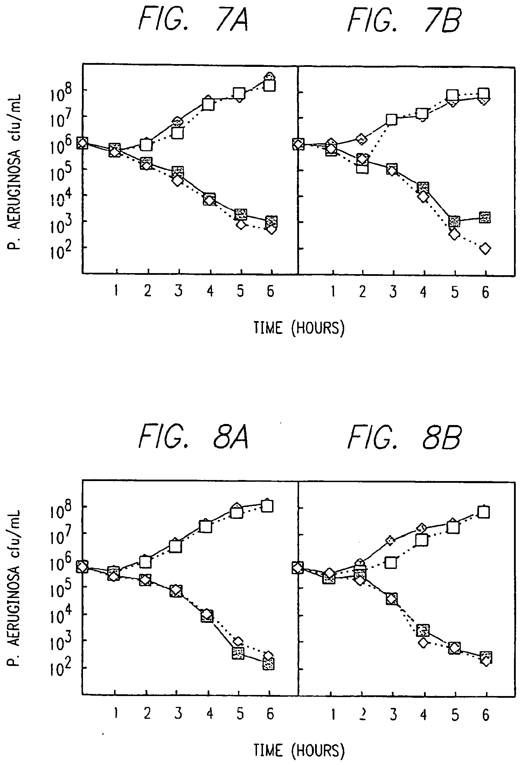

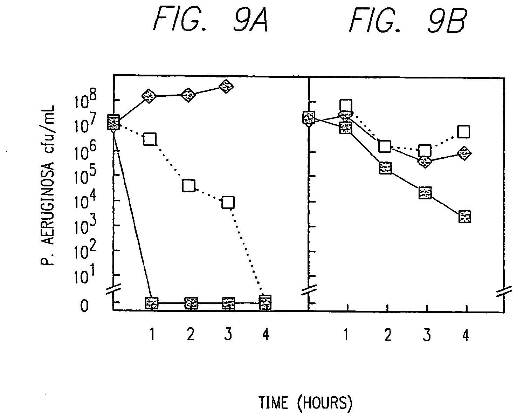

Results of these studies are described in Figures 7 to 9 which represent antibiotic killing curves obtained with different concentrations of the antibiotics aztreonam (Figs. 7 and 8) and tobramycin (Fig. 9), in the presence or absence of mucin or CF sputum. Mucin is a model for the protein binding component of sputum.

Figure 7 illustrates aztreonam activity against *P. aeruginosa* in the absence (Figure 7A) or presence (Figure 7B) of hog gastric mucin. Aztreonam was added to yield a final concentration in the following multiples of the MIC: 0.0 (◆); 0.1 (□); 1.0 (■) ; and 10 (◊).

As seen in Figure 7, the curves without hog gastric mucin (Figure 7A) and with hog gastric mucin (Figure 7B) are virtually identical, indicating no measurable inhibition of the antibiotic by mucin.

Figure 8 shows aztreonam activity against *P. aeruginosa* in the presence or absence of cystic fibrosis (CF) sputum. Aztreonam was added to yield a final concentration in the following multiples of the MIC: · 0.0 (◆); 0.1 (□); 1.0 (■); and 10 (0).

As seen in Figure 8, the curves without CF sputum (Figure 8A) and with sputum (Figure 8B) are virtually identical, indicating no measurable inhibition of the antibiotic by CF sputum.

Tobramycin, which is known to bind mucins and to be inhibited by sputum and mucin, was tested with or without mucin in the same assay for comparative purposes.

Figure 9 shows tobramycin activity against *P*. *aeruginosa* in the absence (Figure 9A) or presence (Figure 9B) of added mucin. Tobramycin was added to yield a final concentration in the following multiples of the MIC: 0.0 (◆); 1.0% (□); and 10% (■).

Figure 9 demonstrates the ability of hog mucin to inhibit the activity of tobramycin. In the absence of mucin, tobramycin killed *P. aeruginosa* effectively, reducing colony counts by seven logs in one hour when applied at 10xMIC. In contrast, the same concentration of tobramycin in the presence of mucin caused much less killing: negligible amounts at one hour and only three to four logs at four hours. At 1xMIC, tobramycin killed seven logs of *P. aeruginosa* in four hours in the absence of mucin, but killed less than one log at four hours in the presence of mucin.

Neither CF sputum nor hog gastric mucin showed significant inhibition of the activity of aztreonam under the conditions of this assay. The *P*. *aeruginosa* killing curves obtained were virtually identical to controls lacking sputum or mucin. Growth of *P. aeruginosa* occurred, as expected, when aztreonam was added in quantities less than the MIC (upper curves in Figures 7-9), while effective killing occurred when aztreonam was present at or above the MIC (lower curves).

This contrasts with the result for tobramycin, an antibiotic known to be inhibited by CF sputum and hog gastric mucin. Addition of mucin to tobramycin resulted in decreased killing by up to four logs, depending on timing and the concentration of antibiotic used. These results confirm the validity of the mucin inhibition assay as a model for interpreting expected outcomes in the lungs of CF patients.

These results show that aztreonam is not inhibited by sputum of cystic fibrosis patients and that it will not be inhibited as a primary or a secondary complementary treatment when administered by inhalation, at least not to the extent that tobramycin is. This implies that aztreonam may be preferable to tobramycin in the treatment of respiratory infections in cystic fibrosis or other patients, as more antibiotic will be available to eradicate *Pseudomonas aeruginosa.*

### VI. Treatment of Pulmonary Bacterial Infections

We describe herein an efficacious treatment and prevention of acute and chronic pulmonary bacterial infections caused by *Pseudomonas aeruginosa, Escherichia coli, Klebsiella pneumonia, Pseudomonas aeruginosa, Haemophilus influenzae, Proteus mirabilis, Enterobacter* species and *Serratia marcescens,* as well as infection caused by antibiotic resistant strains *Burkholderia*. *cepacia, Stenotrophomonas maltophilia, Alcaligenes xylosoxidans,* and multidrug resistant *Pseudomonas aeruginosa.*

### A. Two Modes of Inhalable Treatment

A method for treatment of pulmonary infections comprises administration of aztreonam lysinate in inhalable form whether by aerosol or as a dry powder, several times a day. The aztreonam lysinate daily dose is between 1 and 500 mg/day, with exceptional dose up to 750 mg/day administered in from 1-50 mg/mL for aerosol and from 2 to 200 mg daily dose of dry powder administered in a dose of 1-100 mg/one treatment. The aztreonam lysinate dosage and dosing frequency depends on the type of bacterial infection, severity thereof, age of the patient, the conditions of the patient, etc. In case of cystic fibrosis patients where the lung air capacity is diminished, the dosing is more frequent with lower doses.

The dry powder formulation suitable for treatment of pulmonary infections comprises 1 to 200 mg, preferably about 10 to 100 mg, of powder in an amorphous or crystalline state in particle sizes between 1 and 5 microns in mass median average diameter necessary for efficacious delivery of aztreonam lysinate into the endobronchial space. The dry powder formulation is delivered one to four or more times daily, preferably twice daily. The dry powder formulation is temperature stable and has a physiologically acceptable pH of 4.2-7.5, preferably 5.5 to 7.0, and an over five year long shelf life.

### B. Treatment of Infections in Patients with Suppurative Pulmonary Diseases

Aerosol therapy is particularly useful for treatment of patients suffering from suppurative pulmonary diseases and is especially suitable for treatment of patients with cystic fibrosis, bronchiectasis and those patients on the mechanical ventilation.

Previously, aerosol therapy for cystic fibrosis inhaled (ATCF) antibiotics have demonstrated significant benefit of such treatment to cystic fibrosis (CF) patients suffering from chronic pulmonary infections.

In the US, the most widely used and successful agent in this regard has been tobramycin, which has been shown to produce substantial improvements in lung function and other clinical parameters.

It has now been discovered that inhalable aztreonam lysinate provides successful treatment in cystic fibrosis, bronchiectasis or other suppurative pulmonary disease for pulmonary infections caused by gram-negative bacteria and particularly those caused by antibiotic resistant *Burkholderia cepacia, Stenotrophomonas maltophilia, Alcaligenes xylosoxidans* and multidrug resistant *Pseudomonas aeruginosa.*

Treatment of these multi-resistant bacterial infections with aerosolized aztreonam lysinate has been successful in eradication of the bacteria as described in Example 2.

Such treatment is either stand alone or may be complementary treatment to other antibiotics, such as tobramycin, which upon extended use, results in the development of anti-tobramycin resistance. When the treatment with tobramycin is interspaced with periods of treatment with aztreonam lysinate, such resistance either does not develop or recedes.

### C. Limitations of Current Aerosolized Antibiotics in Treatment of Cystic Fibrosis

To date, an aminoglycoside tobramycin is the only antibiotic with FDA approval for administration as an aerosol. However, despite the benefits obtained in cystic fibrosis patients with administration of aerosolized tobramycin, its utility is somewhat limited.

First, frequent use of aminoglycosides to control pulmonary exacerbations leads to selective development of resistant *Pseudomonas aeruginosa* strains. The widespread emergence of such organisms is acknowledged as a growing crisis in the CF community. For example, 21 % of patients screened from 69 different CF centers for the phase III tobramycin clinical trials had isolates resistant to tobramycin (MIC > 16 µg/mL). Accordingly, many clinicians are reluctant to prescribe this aerosolized aminoglycoside as chronic suppressive therapy, fearing that it could further promote resistance and thus diminish the effectiveness of IV therapy. In order to reduce the risk of such treatment-emergent resistance, tobramycin therapy is restricted to cycles of 28 days on and 28 days off the drug.

A second limitation of aerosolized tobramycin is its lack of activity against several intrinsically tobramycin resistant bacteria, including *Stenotrophomonas maltophilia, Alcaligenes xylosoxidans,* and *Burkholderia* cepacia, the latter of which is widely recognized as a significant threat to cystic fibrosis patients. Cystic fibrosis patients infected with *Burkholderia cepacia* have an increased rate of mortality, and many experience a rapid fatal course, as described in Am. J. Respir. Crit. Care Med., 160:1572-1577, (1999). Additionally, *Burkholderia cepacia* is a transmittable infection which can cause epidemic spread among cystic fibrosis patients. Therefore, a patient infected with *Burkholderia cepacia* must be isolated from other patients.

Aerosolized aztreonam lysinate does not induce resistance to aminoglycosides and has good activity against resistant pathogens observed in cystic fibrosis patients.

An aerosolized aztreonam lysinate can either replace tobramycin, or be used as an alternative and intermittent treatment for tobramycin during the 28-day tobramycin free periods, which are required to prevent development of permanent resistence to tobramycin.

Aztreonam lysinate is an antibiotic with excellent activity against many aerobic gram-negative bacteria, including multi-resistant *Pseudomonas aeruginosa.* The spectrum of activity of aztreonam lysinate is similar to that of the aminoglycoside antibiotics tobramycin and gentamycin, and its antipseudomonal activity is comparable to ceftazidine and in several aspects, it is better than tobramycin. For example, aztreonam lysinate is not inhibited by CF patient sputum, making it much more potent drug than tobramycin which is so inhibited.

Aztreonam lysinate resists destruction by most bacterial β-lactamases, which are the source of much treatment-emergent resistance to β-lactam antibiotics frequently appearing among hospitalized patients.

Aztreonam lysinate's activity against gram-negative bacteria, especially *Pseudomonas aeruginosa*, combined with its excellent safety profile makes it a good alternative to aminoglycosides in the treatment of chronic pulmonary infections among cystic fibrosis patients. Thus far, clinical use of aztreonam lysinate in CF patients has included IV administration of aztreonam as single agent therapy or in combination with other antibiotics for treatment of pulmonary exacerbations.

### D. Advantages of Aztreonam Lysinate as an Aerosolized Antibiotic

Aztreonam lysinate possesses several features that make it very attractive for aerosol administration to CF patients.

The first of these features stems from its mechanism of action, which, unlike aminoglycoside antibiotics, involves preferential binding to penicillin binding protein 3 (PBP3) and subsequent interference with bacterial cell wall synthesis. Because aztreonam lysinate's mechanism of action differs from that of tobramycin, its use does not contribute to emergence of aminoglycoside-resistant strains of *Pseudomonas aeruginosa*.

The second advantage of an aerosolized formulation of aztreonam lysinate is its activity against tobramycin resistant, and multidrug resistant *Pseudomonas aeruginosa*. When isolates from patients enrolled in the Phase II tobramycin trials were examined, nearly 75% of isolates with a tobramycin MIC >16 µg/mL were susceptible to aztreonam lysinate.

The third feature is aerosolized aztreonam lysinate ability to control intrinsically tobramycin resistant organisms, especially *Burkholderia cepacia,* which is considered resistant to the levels of aztreonam lysinate achieved by parenteral administration.

### VII. Antibacterial Activity of Aztreonam

In order to test antibacterial activity of aerosolized aztreonam against multi-resistant strains *of Pseudomonas aeruginosa, Burkholderia cepacia, Stenotrophomonas maltophilia* and *Alcaligenes xylosoxidans,* the *in vitro* activities of aztreonam in concentrations corresponding to those achievable with inhalable aztreonam were tested against clinical isolates from cystic fibrosis patients.

The aztreonam aerosol delivery according to the invention achieves concentrations of aztreonam to reach levels from 500 to as high as 8000 µg/mL, with an average level around 2,000 µg/mL, of aztreonam in the sputum. These levels depend on the formulation as well as on the nebulizer used for aerosolization. With certain nebulizers the concentration of aztreonam can reach an average level of 5,000 µg/mL.

*In vitro* determined susceptibilities of the tested bacteria is predictive of clinical efficacy of inhaled aztreonam aerosol or dry powder.

Aztreonam kills by lysing cell walls as long as the local concentration of antibiotic exceeds the bacteria minimal inhibitory concentration (Med. Clinics N. Am., 79: 4, 733-743, (1995)).

The *in vitro* activity of high aztreonam concentrations against clinical isolates of *B. cepacia, S. maltophilia* and *A. xylosoxidans* was tested at the Children's Hospital and Regional Medical Center in Seattle, WA. Testing was performed on broth microdilution trays made with 2 fold concentrations of aztreonam from 2 to 2048 µg/mL. *Staphylococcus aureus,* a gram positive organism, was used as a negative control.

Detailed procedure used for testing is described in Example 1. Results are seen in Table 9.

**Table 9**

| Organism (# of isolates) | MIC Range | MIC50 | MIC90 |
|---|---|---|---|
| P. aeruginosa (54) | 2-1024 | 16 | 512 |
| B. cepacia (38) | 2-2048 | 32 | 512 |
| S. mallophilia (20) | 8->2048 | 256 | >2048 |
| A. xylosoxidans(20) | 2>2048 | 256 | 2048 |
| S. aureus(20) | 512-2048 | 1024 | 2048 |

For testing, each microwell plate contained a 2-fold dilution, 2, 4, 8, 16, 32, 64, 128, 256, 512, 1024 and 2048 of aztreonam. Each plate containing the microwells was used to test one isolate of one organism.

Table shows the different species of bacteria tested for sensitivity, that is the ability of the antibiotic to inhibit its growth, to aztreonam, with the number of isolates for each species given in parenthesis. The column designated "MIC range" shows the range of the lower and upper limits of sensitivities seen in the tested isolates.
The column designated MIC50 shows the median level of sensitivity for the most sensitive 50% isolates. The final column, designated MIC90, shows the median value for the level of sensitivity for the most sensitive 90% of the isolates.

Table 9 shows results of comparative *in vitro* activity of aztreonam against clinical isolates obtained from cystic fibrosis patients.

For interpretation of this data, the values which represent what concentration of aztreonam is required to inhibit growth of bacteria are compared with the concentrations of aztreonam obtainable by the different routes of administration. Thus, for intravenous administration of aztreonam, the serum level following administration of 2g of aztreonam, the maximum allowed intravenous dose, the serum level peak is 256µg/mL and then declines rapidly. At six hours following the administration, the aztreonam level in the serum is in the range of 16µg/mL. For safety reasons, intravenous aztreonam arginine can only be administered every six hours. With the possible exception of *Pseudomonas aeruginosa* that has a MIC50 of 16µg/mL, all other organisms would be predominantly resistant to intravenous aztreonam, as their level of resistance exceeds even the peak concentration (256 µg/mL) of serum concentration of sputum of aztreonam following intravenous administration. Since, however, the bacteria resistance is relative to drug concentration, for aerosol administration, the peak concentration should be at least in the 500 to 2000 µg/mL range. Such range is achieved with the doses of aztreonam and the formulation combined with the efficient nebuliser. At the 500-2000 µg/mL concentration in the sputum, the aerosol therapy is able to treat most endobronchial infections caused by gram-negative bacteria, specifically those bacteria listed in Table 9, with exception of *Staphyloccocus aureus*.

The MIC50 and MIC90 have shown that treatment of *P. aeruginosa* with inhalable aztreonam eradicates most *P. aeruginosa* isolates with the high concentrations of aztreonam in sputum of cystic fibrosis patients obtainable after aerosol delivery. The data obtained for *Burkholderia cepacia* isolate indicated that at least half of patients would be expected to respond to such treatment with eradication of the bacteria. If sufficiently high concentrations of aztreonam are delivered to the lung, the percentage is expected to be higher. Since the *Burkholderia cepacia* infection is now viewed as a largely untreatable condition, treatment with inhalable aztreonam by aerosol is the first documented efficacious therapy.

The results obtained in these studies are surprising and unexpected as there is no indication in the literature that *Burkholderia cepacia* is susceptible to treatment with aztreonam. The data also shows that some isolates of *S. maltophilia* and *A. xyloxidans* respond to high concentration of aztreonam.

Inhalation of aztreonam as described permits reaching concentrations of aztreonam in the sputum as high as 2000-5,000 µ/mL. The sputum aztreonam levels achieved via aerosol administration exceed those required to inhibit organisms responsible for otherwise untreatable infections in CF patients.

Furthermore, aztreonam delivered by inhalation to all patients with *Burkholderia cepacia and*/*or S. maltophilia and*/*or A. xyloxidans* together with other antibiotics whether administered systemically parenterally or by inhalation contributes to synergy of such treatment. A combination of inhalable aztreonam with other antibiotics provides another therapeutic approach to treat multi-resistant bacterial strains.

The studies described herein demonstrated that the concentrations of aztreonam achieved following aerosol administration have activity against *Burkholderia cepacia* isolated from CF patients' sputum as well as against other bacteria which are largely resistant to treatment with other antibiotics.

The MIC50 and MIC90 observed for a gram positive bacteria, *Staphylococcus aureus*, show that high concentrations of aztreonam had some activity against this gram positive bacteria. These findings, however, have no great significance as there are many other drugs with reasonable efficacy against *Staphyloccocus aureus*.

### VIII. Safety and Clinical Testing

The infections requiring particular attention are infections caused by and include *B. cepacia*, *S. maltophilia* and *A. xylosoxidans*, as well as multi-resistant strains of *Pseudomonas aeruginosa.* The most clinical significant infection is the former.

In order to determine if an appropriately formulated aztreonam lysinate for aerosolization could become effective for treatment of these rare but very resistant bacterial strains, the treatment with aerosolized aztreonam lysinate was initiated and tested in a cystic fibrosis patient having a severe *Burkholderia cepacia* infection which did not respond to any treatment. The clinical treatment and results obtained with an aerosolized aztreonam lysinate is described in Example 2.

Safety of the aztreonam lysinate formulation was also studied both in man and in Beagle dog. Conditions of these studies are described in Samples 11 and 12.

Results of both studies confirm the safety of the aztreonam lysinate formulation for inhalation. As compared to a formulation containing arginine, the new formulation is safe in man (Example 10) and in dog at up to 200 fold of the human dose shown in a 28 day dog study (Example 11). Increased safety establishes utility of the aztreonam lysinate in both instances.

Safety results from both studies show that there were no serious adverse events recorded during the trial and no subject was withdrawn from the trial because of an adverse event. In total, 7 post-dose adverse events were reported for 7 subjects. No single adverse event was experienced by more than one subject. A single drug-related adverse event occurred in each of the 95 and 190 mg inhaled aztreonam dose groups (headache and dizziness, respectively) and 2 drug-related adverse events occurred in the 285 mg inhaled aztreonam dose group (dysgeusia, i.e. unpleasant taste and cough). One adverse event was of Grade 2 severity (headache) and the remaining adverse events were of Grade 1 severity. All adverse events resolved before the end of the trial. The adverse event of cough led to discontinuation of the trial medication, although the subject continued in the trial and completed all trial assessments.

There were no notable mean changes from baseline in any post-dose pulmonary function parameter. One subject, who was dosed with placebo, had an FEV₁ decrease from baseline of greater than 15% (+30 min). This was recorded as an adverse event, but was not considered to be related to the trial medication.

There were no notable mean changes from baseline in any hematology or coagulation parameter assessed.

There were no notable mean changes from baseline in systolic and diastolic pressure, pulse rate, oral temperature, respiration rate or pulse oximetry in subjects dosed with placebo or 90 mg, 190 mg or 285 mg inhaled aztreonam. No individual subject value in any of these parameters was reported as an adverse event.

There were no notable mean changes from baseline in any ECG parameter assessed and no individual subject ECG value was reported as an adverse event. No changes from baseline were noted on any post dose physical examination.

In conclusion, inhaled aztreonam was generally safe and well tolerated when administered at does of 95 mg, 190 mg and 285 mg in this trial.

There were no clinically significant changes in FEV₁ (defined as a decrease from baseline of 15% or more) in any subject treated with aztreonam. One subject who was treated with placebo experienced a decrease from baseline in FEV₁ of 15.58%. This was reported as an adverse event not considered to be related to treatment. There were no clinically significant changes in any other safety measurement (in either mean or individual values) there were considered to be treatment-related.

The objective of the second study was to assess the tolerability and toxicity of aerosolized aztreonam lysinate formulation in the Beagle dog after 28 day repeat dosing by the inhalation route and to evaluate the reversibility of any effects after a 14 day recovery period. Inhalation exposure was undertaken using a closed face mask system with the dogs breathing passively from an ultrasonic nebulizer.

Conditions under which the study was conducted are described in Example 11.

Overall results of this study show that the inhalation of nebulized aztreonam lysinate is safe and there were no observed adverse clinical signs or treatment related effects on body weight, food consumption, ophthalmoscopic findings, ECG readings, laboratory investigations or organ weights.

There were no necropsy or histological findings that could be attributed to treatment with Aztreonam. Since the anticipated human dose is 75 mg, and the average weight is 75 kg, the safety margin may be as high as 200 fold over the human dose.

### UTILITY

The method of treatment and the inhalable aztreonam lysinate compositions disclosed herein is suitable for treatment of respiratory tract infections caused by *Burkholderia cepacia, Stenotrophomonas maltophilia, Alcaligenes xylosoxidans,* and multidrug resistant *Pseudomonas aeruginosa* as well as for treatment of other pulmonary infections caused by gram-negative bacteria.

### REFERENCE EXAMPLE 1

### In vitro Testing of Isolates from Cystic Fibrosis Patients

This example describes procedure used for in vitro studies of bacterial isolates obtained from cystic fibrosis patients.

Bacterial respiratory tract isolates (144) from patients with CF that had been stored at -70°C were cultivated by two consecutive overnight passages at 37°C on 5% blood agar (Remel, Lenexa, KS).

Minimal inhibitory concentrations (MIC's) were determined by the following steps:
MIC Antimicrobial Testing Aerobic organisms
   1. MIC trays were brought to room temperature.
   2. 3.0 mL physiological saline was inoculated with an 18-24h culture of organism to be tested to a turbidity equal to a 0.5 McFarland Standard (1.5 x 10⁸ CFU/mL). This corresponds to an OD600 of 80 - 88% transmission.
   3. Within 15 minutes of preparation, the adjusted inoculum suspension was diluted by transferring 100 mL into a 2.9 mL diluent of sterile water.
   4. The suspension was gently mixed by inversion and 10 mL was dispensed into each MIC well having initial volume of 100 µl. The final concentration in each well was equal to 5 x 10⁵ CFU/mL or 5 x 10⁴ CFU/well.
   5. Trays were incubated aerobically at 37°C for 16-20 hours. The same incubation temperature was maintained for all cultures. Microdilution trays were not stacked more than four high.
   6. Antimicrobial endpoint was read and recorded as the first well showing no readily visible growth or haze as detected by the unaided eye.
   7. The microdilution trays were contacted with 2 fold concentrations of aztreonam lysinate from 2 to 2048 mg/mL. Each microwell plate was treated with a 2-fold dilution of aztreonam lysinate in following amounts: 2, 4, 8, 16, 32, 64, 128, 256, 512, 1024 and 2048 µg/mL. Each plate containing the microwells was used to test one isolate of one organism.
   8. Results were read and recorded.

### REFERENCE EXAMPLE 2

### Clinical Treatment of Patient with Burkholderia cepacia

This example describes a first finding of efficacy of the aerosolized aztreonam treatment of a cystic fibrosis patient suffering from resistant *Burkholderia cepacia*.

The patient was a 20-year-old female with cystic fibrosis and end stage lung disease. She had been diagnosed with *Burkholderia cepacia* pulmonary infections that had become resistant to all known intravenous, oral and inhaled antibiotics. She had two-documented genetically different strains of *Burkholderia cepacia*. For this reason the patient was rejected as a candidate for a lung transplant.

The patient was provided with a formulation of the invention comprising 200 mg/mL of aztreonam and instructed to use this formulation in 3 to 5 mL of diluent and use it in an air compressor powered breath enhanced jet nebulizer and take the therapy twice a day. This type of nebulizer only delivers about 10 to 20% of the dose placed in the nebulizers to the lungs, however, that was only nebulizer available to the patient for home treatment.

After three months of continuous twice a day therapy, the pulmonary infection was successfully treated and no evidence of *Burkholderia cepacia* could be detected. The patient was considered treated from the infection and eventually underwent a successful lung transplant procedure.

There was no postoperative reoccurrence or relapse of the *Burkholderia cepacia* infection despite of intensive immunosuppression therapy following the transplantation.

These findings were surprising since previous use of commercially available aztreonam arginine in an older generation delivered in even less efficient nebulizers did not lead to eradication of *P. aeruginosa* as described in Clinics Chest Med., 19:473-86, (Sept. 1998). In the trial described there, the authors stopped therapy at the development of any aztreonam resistance rather than continuing treating these patients. Prior work did not test or speculate that this therapy could be effective in treating other gram negative bacteria including *Burkholderia cepacia, S. maltophilia, X. xylosoxidans,* or other multidrug resistant pseudomonas infections.

The results obtained with treatment of the above patient are even more surprising in that the eradication of *Burkholderia cepacia* is extremely rare occurrence, particularly when the infection is well established as was in the case of this patient.

### EXAMPLE 3

### Preparation of Aztreonam Lysinate Dry Powder

This example provide methods and procedures used for preparation of aztreonam lysinate containing inhalable dry powder.

For dry powder formulation, a purified aztreonam lysinate is milled to a powder having mass median average diameters ranging from 1 to 5 µ by media milling, jet milling, spray drying, or particle precipitation techniques.

Particle size determinations is made using a multi-stage Anderson cascade impactor.

Media milling may be accomplished by placing the drug into a mill containing, for example, stainless steel or ceramic balls and rotating or tumbling the material until the desired drug particle size ranges are achieved.

Jet milling uses very high pressure air streams to collide particles with one another, with fine particles of the desired size being recovered from the mill.

Spray drying is achieved by spraying a fine mist of drug solution onto a support and drying the particles. The particles are then collected.

Particle precipitation is achieved by adding a co-solvent to spray dried particles. The solubility of the drug falls to the point where solid drug particles are formed. The particles are collected by filtration or centrifugation. Precipitation has the advantage of being highly reproducible and can be performed under low temperature conditions, which reduce degradation.

### EXAMPLE 4

### Dry Powder Inhalers

Metered dose and the dry powder formulations prepared according to the invention may be used directly in metered dose or dry powder inhalers.

A metered dose inhaler consists of three components: a canister containing the propellant drug suspension, a metering valve designed to deliver accurately metered volumes of the propellant suspension, and an oral adapter which contains a spray orifice from which the metered dose is delivered. In the rest position, the metering chamber of the valve is connected to the drug suspension reservoir via a filling groove or orifice. On depression of the valve this filling groove is sealed and the metering chamber is exposed to atmospheric pressure via the spray orifice in the oral adapter and the valve stem orifice. This rapid pressure reduction leads to flash boiling of the propellant and expulsion of the rapidly expanding mixture from the metering chamber. The liquid/vapor mixture then enters the expansion chamber which is constituted by the internal volume of the valve stem and the oral adapter. The mixture undergoes further expansion before being expelled, under its own pressure, from the spray nozzle. On exit from the spray orifice, the liquid ligaments which are embedded in propellant vapor are torn apart by aerodynamic forces. Typically, at this stage, the droplets are 20 to 30µ in diameter and are moving at the velocity of sound of the two-phase vapor liquid mixture (approximately 30 meters per second). As the cloud of droplets moves away from the spray nozzle, it entrains air from its surroundings and decelerates, while the propellant evaporates through evaporation and the entrained droplets eventually reach their residual diameter.

At this point, the particles/droplets consist of a powdered drug core coated with surfactant. Depending on the concentration and the size of the suspended material the powdered drug core consists of either individual drug particles or aggregates. Currently, meter dose inhaler technology is optimized to deliver masses of 80 to 100 micrograms of drug, with an upper limitation of 1 mg of drug deliverable.

An alternated route of dry powder delivery is by dry powder inhalers. There are two major designs of dry powder inhalers, device-metering designs in which a reservoir of drug is stored within the device and the patient "loads" a dose of the device into the inhalation chamber, and factory-metered devices in which each individual dose has been manufactured in a separate container. Both systems depend upon the formulation of drug into small particles of mass median diameters from 1 to 5 microns, and usually involve co-formulation with large excipient particles (typically 100 micron diameter lactose particles). Drug powder is supplied into the inhalation chamber (either by device metering or by breakage of a factory-metering dosage) and the inspiratory flow of the patient accelerates the powder out of the device and into the oral cavity. Non-laminar flow characteristics of the powder path cause the excipient-drug aggregate to decompose, and the mass of the large excipient particles causes their impaction at the back of the throat, while the inhaler drug particles are deposited deep in the lungs. Current technology for dry powder inhalers is such that payload limits are around 50 mg of powder (of which drug is usually a partial component by mass). Excipients commonly used are lactose, however in the current case aztreonam is reacted with amino acid lysine and such reaction leads to a better powder formation and more stable powder formulation.

Effective dosage levels of aztreonam lysinate antibiotic for dry powder inhalation and metered dose inhalation result in the delivery of at least about 25 mg, and more preferable about 50 to about 100 mg of aztreonam lysinate to the lung of the patient receiving treatment. Depending on the efficiency of the dry powder delivery device, dry powder formulations suitable for use in the invention comprise from about 1.0 to about 250 mg, preferably from about 10 to about 100 mg of powder in an amorphous or crystalline state in particle sizes between 1 and 5 microns in mass median average diameter necessary for efficacious delivery of the antibiotic into the endobronchial space.

### EXAMPLE 5

### Preparation of Aztreonam Lysinate Powder

This example describes procedure used for preparation of aztreonam lysinate powder.

To a solution of 10 g (23 mmol) of aztreonam lysinate in 100 mL of MeOH cooled in an ice bath was added dropwise 23 mL (23 mmol, 1.0 eq) of 1N sodium hydroxide solution. The resulting solution was warmed to ambient temperature over a period of 30 min, and then the solvent was removed under reduced pressure. Diethylether (50 mL) was added and the slurry concentrated. This step was repeated four times to provide a yield of 10.1 g (96%) of aztreonam lysinate salt as a white powder.

### EXAMPLE 6

### Formulation and Spray Drying of Aztreonam Lysinate

Aztreonam (α-aztreonam, 29.4 g with 15% moisture, equivalent to 25.0 g anhydrous) was suspended and rapidly stirred in water (190 mL) and cooled with a crushed ice bath. L-Lysine (anhydrous, 17.7 g, dissolved in 40 mL of room temperature water) was titrated over 6 minutes to the milky white suspension to obtain a pH of 4.34. The total volume of the aztreonam lysinate solution was approximately 270 mL and had a yellow to light brown color. Approximately 1 g of charcoal was added to the stirring solution and was then filtered. The aztreonam lysinate solution was kept at 2 to 10°C. Spray drying was accomplished giving a yield of 22.2 g (56%) of aztreonam lysinate. Below illustrates an unoptimized method for spray drying:
Inlet Set 135°C.
Aspirator 90% (a value of 100% = 35 cubic meters/hr).
Pump 34% (a value of 100% = 1500 mL/hr).
Ar flow at nozzle 400 L/hr initial; at middle of run increased to 600 L/hr.
Receiver flask temp 35 to 40°C.

### REFERENCE EXAMPLE 7

### Testing Nebulizers

This example describes testing of nebulizers in clinical conditions to determine dose to be used in each.

A clinical study is conducted in order to determine the concentration of aztreonam lysinate in the aerosol formulation required to achieve a sputum concentration between 500µg/gm and 2000 µg/gm sputum at 10 min post-completion of aerosol administration using an atomizing, ultrasonic or jet nebulizer.

In this study, cystic fibrosis patients receive serial escalating doses of multiple of 75 mg aztreonam (1 mL of a 75 mg/mL solution in 1/4 NS) from each of the nebulizers. The doses are separated by at least 2 days and not more than 5 days. Peak serum and sputum concentrations are assessed.

### REFERENCE EXAMPLE 8

### Testing of Sputum Inhibitory Activity

This example describes conditions used for testing inhibitory activity of aztreonam lysinate and tobramycin on sputum or hog gastric mucin.

### Reagents

Unless stated otherwise, all chemicals were purchased from Sigma Chemical Company (St. Louis, Mo.), and all solutions were prepared in sterile deionized water. Aztreonam (Azactam^{®}) were obtained from Elan Biopharmaceuticals. Aztreonam lysinate was prepared at Corus Pharma, Seattle, WA. Working stock solutions of aztreonam and aztreonam lysinate were prepared in sterile deionized water and used immediately.

### Culture medium

Divalent cation adjusted Mueller Hinton broth (CAMHB) was purchased from PML and used both as the study growth medium for *P. aeruginosa* and as the assay growth medium.

### Sputum

Sputum was obtained from children and adults with CF who were not receiving any other antimicrobial drug for at least 48 hours prior to the collection of the sample. Sputum was sterilized by stirring with a magnetic stirrer under UV light for 4 hours. Sterility was tested by inoculating 100 uL of sputum into 10 mL of CAMHB a row medium and incubating overnight. Resulting culture was examined for turbidity and 100 muL were plated on Luria agar to ensure sterility. The sputum samples were kept frozen at -20°C until used.

### Organisms

Fresh subcultures of *P. aeruginosa* strain PA27853 were used for each experiment. Freezer stock was grown on Luria agar plates (Sigma L-3522) overnight at 37°C. A single colony was picked and inoculated into 5 mL of CAMHB and grown for 16 hours at 37°C with shaking at 250 rpm. This overnight culture was diluted 1:10,000 in fresh CAMHB or in fresh CAMHB supplemented with 10% (w/v) porcine gastric mucin (Sigma M-1778), then autoclaved, or 1% sterilized CF sputum.

### Killing curves

*P. aeruginosa* (initial density ∼10⁶ CFU/mL) was grown in overnight culture and diluted 1:10,000 in broth. The dilutions were each divided into 4 tubes (10 mL per tube) and antibiotic was added to each tube to a final concentration of 0, 0.1, 1, and 10 times the MIC for strain PA27853 (4 µg/mL for aztreonam, 1.56 µg/mL for tobramycin, determined by standard methods). Each tube was incubated at 37°C with 250 rpm shaking. Each hour, samples were removed from the tube, diluted, and plated on Luria agar for quantitation. Plates were incubated overnight at 37°C and colonies were counted by hand.

### REFERENCE EXAMPLE 9

### Clinical Trial Protocol

This example describes a protocol used for clinical trial and to compare the pharmacokinetics of increasing dosage of an aztreonam lysinate formulation administered by the PARI electronic nebulizer to patients with cystic fibrosis.

The primary aim of this study was to determine which of the tested dose levels delivered by aerosol can deliver sufficient amount of aztreonam lysinate to achieve a mean peak sputum aztreonam lysinates concentration of 1000 µg/gm or greater measured 10 minutes after the completion of nebulization in patients with CF.

The secondary aim was to determine whether the aztreonam lysinate concentration required to achieve a mean peak sputum concentration of 1000 µg/gm or greater is safe and well tolerated by the patient.

### Study Design

This was an open label, multicenter, randomized, dose escalation study.

Each arm contained different dose. Two arms delivered the same aztreonam lysinate formulation.
1. 1.0 mL of aztreonam lysinate solution of 75 mg/mL
2. 2.0 mL of aztreonam lysinate solution of 75 mg/mL
3. 3 . 0 mL of aztreonam lysinate solution of 75 mg/mL

### Efficacy and Safety Assessment

In this study, the following efficacy and safety parameters that were assessed were:

The efficacy was determined for each nebulizer by measuring concentration of aztreonam lysinate in sputum 10 minutes after completion of nebulization. Mean concentration of 1000 µg/gm of sputum was considered adequate.

The safety parameters assessed:
1. Incidence of treatment related adverse reactions occurring during the administration of the aerosolized aztreonam lysinate at the different dose levels.
2. Acute bronchospasm at the time of drug administration.
3. Absorption of aztreonam lysinate into the systemic circulation.

Each patient received in random order at least one administration. Each aerosol administration was separated by a minimum of 48 hr. Sputum samples were collected at baseline, 1, 2, 4 and 6 hours post-completion of the aerosol drug administration to measure aztreonam lysinate concentration. Serum samples were collected at baseline, 1, 2, 4 and 6 hours post-completion of aerosol administration to measure aztreonam lysinate levels.

Airway irritation and acute bronchospasm were assessed by measuring spirometry immediately prior to and 30 min post-completion of aerosol administration. A decrease in forced expired volume in one second (FEV1) >15% in the 30 min spirometry test is considered evidence of bronchospasm.

Additional objectives of this study were to determine and at what dose the PARI electronic nebulizer tested can aerosolize sufficient aztreonam lysinate sulfate to achieve a mean peak sputum aztreonam lysinate concentration of 1000 µg/gm or greater in at least 85% of patients with CF measured 10 minutes after the completion of nebulization to determine whether the aztreonam lysinate concentration required to achieve a mean peak sputum concentration of 1000 µg/gm or greater is safe and well tolerated by the patient. Safety is defined as a lack of acute bronchospasm and minimal systemic absorption.

### Patient Treatment

All patients with underlying disease of cystic fibrosis (CF), confirmed at entry by the inclusion/exclusion criteria specified in this protocol, were eligible for enrollment into the study. Investigators at the participating CF centers selected patients that meet all of the inclusion criteria and one of the exclusion criteria.

Eligible patients were admitted to the study center on the day of the study and receive aerosol therapy if they fulfilled entrance criteria.

Physical exam is administered by a physician or RC nurse prior to initial aerosol treatment only.

Vital signs, height, weight, oximetry, assessment of current respiratory status and brief medical history were used. Sputum and serum samples were collected to measure baseline aztreonam lysinate concentrations.

Patients were sitting upright and use nose clips during the aerosol administration.

The total duration of time and the number of inhalations required to complete the aerosol treatment were recorded.

Any evidence of wheezing or respiratory distress are recorded as well as number of rest periods required by the subject because of dyspnea or excessive coughing during the administration period.

Immediately after completing the aerosol therapy, the subject rinsed with 30 mL of normal saline through the mount, gargled for 5-10 seconds and expectorated the rinse. This was repeated for a total of three rinses.

Sputum specimens were collected at 10 minutes after rinsing oral cavity and 2 hours after completion of the aerosol drug administration.

Serum was collected at 1 and 2 hours after completion of the aerosol drug administration for determination of the aztreonam lysinate levels.

Spirometry was obtained 30 minutes following completion of the aerosol drug administration.

Following the last aerosol treatment of the study, patients received a brief physical exam after post-spirometry has been measured.

### REFERENCE EXAMPLE 10

### Safety Clinical Trials

This example describes clinical protocol used for safety clinical trial with aztreonam lysinate.
Name of Finished Product : Aztreonam for Inhalation
Name of Active Ingredient : Aztreonam lysinate

This was a randomized, double-blind, placebo controlled trial to assess the safety and tolerability of inhaled aztreonam lysinate in healthy male and female volunteers.

The primary objective was to determine the safety and tolerability of 3 escalating doses of aztreonam for inhalation in male and female volunteers.

### Methodology

Subjects were screened for inclusion in the trial up to 21 days before dosing and their eligibility was confirmed at the day 1 visit. Subjects were admitted to the clinic in the morning on the day before dosing (Day -1). Within each of the 3 treatments groups receiving 95 mg, 190 mg and 285 mg inhaled aztreonam, subjects were allocated randomly to either active treatment (6 subjects) or to placebo (2 subjects). Progression to the 190 mg and 285 mg doses occurred only when blinded safety data from the 95 mg and 190 mg groups, respectively, had been assessed. On the morning of day 1, subjects self-administered their allocated trial medication by inhalation using an eFlow™IMP nebulizer (PARI). Subjects remained in the clinic for 24 h after dosing and returned 3 days after dosing for a follow-up visit. Safety was monitored throughout the trial.

### Number of Subjects

24 subjects (3 groups of 8 subjects) were recruited and 24 were included in the safety analysis.

### Diagnosis and Main Criteria for Inclusion

Subjects were male or female non-smokers, aged 18 to 55, weighing between 50 and 100 kg with a body mass index of 18 to 28 kg.m⁻², with a negative Coombs' test result and a forced expiratory volume in one second (FEV₁) of at least 80% of the predicted normal.

### Test Product, Dose and Mode of Administration

Placebo (1, 2 or 3 mL sterile 0.9% saline; manufactured by Phoenix Pharma, was self-administered by the subject into the airways using an eFlow™IMP nebulizer (PARI).

### Safety

Adverse events, laboratory data (hematology, clinical chemistry, Coombs' test, coagulation and pregnancy test for women of childbearing potential), urinalysis, vital signs, ECG, physical examination (including chest auscultation) and pulmonary function tests.

### Safety Results

No serious adverse events were recorded during the trial and no subject was withdrawn from the trial because of an adverse event.

### REFERENCE EXAMPLE 11

### Beagle Dog Safety Study

This example describes conditions used for Beagle dog safety studies.

Sixteen male and female Beagle dogs were allocated to 4 dose groups and treated as follows:

| Dose Group/ Treatment | Target Dose Levels (mg . kg⁻¹ . day⁻¹) | | Animal Numbers/Allocation | | |
|---|---|---|---|---|---|
| | Total | Pulmonary | | Males | Females |
| 1 - Vehicle Control | 0 | 0 | Main Study | 1-3 | 17-19 |
| | | | Recovery | 4-5 | 20-21 |
| 2 - Low Dose | 40 | 8 | Main Study | 6-8 | 22-24 |
| 3 - Intermediate Dose | 80 | 16 | Main Study | 9-11 | 25-27 |
| 4 - High Dose | 200 | 40 | Main Study | 12-14 | 28-30 |
| | | | Recovery | 15-16 | 31-32 |

During the pretrial and recovery phases of the study animals were monitored at least once daily for any adverse clinical signs. During the treatment period, all animals were examined for any adverse clinical signs before exposure, continuously during exposure and 1-2 hours after exposure. Body weights were recorded weekly whilst food consumption was monitored daily up until the end of the study period.

Ophthalmoscopic examinations were undertaken once pretrial, during Week 4 of treatment and towards the end of the 14 day recovery period for designated animals. Electrocardiograms were recorded once pretrial, on Days 2 and 28 of treatment and from designated recovery animals towards the end of the 14 day recovery period.

Blood and urine samples for routine hematology, clinical chemistry and urinalysis investigations were obtained from all animals once pretrial, during Week 4 of treatment, and from designated recovery animals towards the end of the 14 day recovery period. Blood samples for toxicokinetic analysis were collected from all animals from Groups 2, 3 and 4 on Days 1 and 27 of exposure at the following target time points: predose, immediately post dose (IPD) and at 0.25, 0.5, 1, 2, 4, 6, 8 and 24 h post dose. Samples were collected from Group 1 animals predose and immediately post dose. Urine samples for toxicokinetic analysis were collected from all animals on Days 1 and 27 of exposure over a 24 h period.

On completion of the 28/29 day treatment period or 14 day recovery period, all animals were subjected to a detailed necropsy with recording of organ weights. Microscopic evaluation was undertaken on a comprehensive list of tissues.

Overall estimated mean achieved doses of 0, 53.0, 94.3 and 194.7 mg. kg⁻¹. day⁻¹ (estimated mean pulmonary doses of 0, 10.6, 18.9 and 38.9 mg.kg⁻¹.day⁻¹) were achieved for Groups 1, 2, 3 and 4, respectively. Particle size distribution measurements indicated the Aztreonam aerosol was respirable for dogs.

Treatment described herein was safe method for any sign of adverse reaction.

### EXAMPLE 12

### Preparation of α-Aztreonam Lysinate Salt

This example describes procedure used for preparation of α-aztreonam lysinate salt.

α-Aztreonam (29.4 g with 15% moisture, equivalent to 25.0 g anhydrous) was suspended and rapidly stirred in water (190 mL) and cooled on a crushed ice bath. L-Lysine (anhydrous, 17.7 g, dissolved in 40 mL of room temperature water) was titrated over 6 minutes to the milky white suspension to obtain a pH of 4.34. The total volume of the aztreonam lysinate solution was approximately 270 mL and had a yellowish color without any particulate matter. Approximately 1 g of charcoal was added to the stirring solution and was then filtered. The α-aztreonam lysinate solution was stored at a temperature of 2°C.

### EXAMPLE 13

### Spray Drying of α-Aztreonam Lysinate

This example describes conditions used for spray drying of α-aztreonam lysinate.

Spray drying of α-aztreonam lysinate was accomplished by spraying a fine mist of α-aztreonam lysinate solution onto a support and drying under vacuum. The dried particles are collected giving a yield of 22.2 g (56%) of α-aztreonam lysinate.
An optimized method for spray drying
Inlet Set 135°C.
Aspirator 90% (a value of 100% = 35 cubic meters/hr). Pump 34% (a value of 100% = 1500 mL/hr).
Ar flow at nozzle 400 L/hr initial; at middle of run increased to 600 L/hr.
Receiver flask temp 35 to 40°C.

## Claims

1. A process for preparing an aztreonam lysinate dry inhalable powder formulation consisting of aztreonam lysinate, the process comprising the steps of:
a) solubilizing the alpha form of aztreonam in water to form a slurry;
b) titrating an aqueous solution of lysine to form aztreonam lysinate in solution; and
c) drying the aztreonam lysinate from the solution by lyophilisation or spray drying; and wherein the aztreonam lysinate is milled, precipitated, spray dried or otherwise processed to a particle size between 1 to 5 microns.

2. The process of claim 1 wherein the milling is accomplished by media milling to a powder having a mass median average diameter from 1 to 5 microns.

3. The process of claim 1 wherein the milling is accomplished by jet milling to a powder having a mass median average diameter from 1 to 5 microns.

4. The process of claim 1 wherein the drying of the aztreonam lysinate is accomplished by spray drying.

5. The process of claim 1 wherein the particle size is accomplished by particle precipitation techniques to a powder having a mass median average diameter from 1 to 5 microns.

6. The process of claim 1 wherein drying the aztreonam lysinate is accomplished by lyophilization.

7. The process of claim 1 wherein the solubilized aztreonam composition is maintained at a pH below 6.

8. The process of claim 1 carried out at a temperature of from 2 to 20°C.

9. The process of claim 8 carried out at a temperature of from 2 to 8°C.

10. The process of claim 7 wherein solubilized aztreonam composition is maintained at a pH between 4.6 and 4.8.

11. The process of any of claims 1 to 10 in which a reservoir of the aztreonam lysinate dry powder formulation is placed in a device suitable to deliver the dry powder directly to the lungs.

12. The process of any of claims 1 to 11 in which an individual dose of the aztreonam lysinate dry inhalable powder formulation is manufactured in a container.

## Patentansprüche

1. Verfahren zur Herstellung einer trockenen, inhalierbaren Aztreonam-lysinat-Pulverzubereitung, die aus Aztreonam-lysinat besteht, wobei das Verfahren die folgenden Schritte umfasst.
a) Solubilisieren der alpha-Form von Aztreonam in Wasser zur Bildung einer Aufschlämmung;
b) Titrieren einer wässrigen Lösung von Lysin zur Bildung von Aztreonam-lysinat in Lösung; und
c) Trocknen des Aztreonam-lysinats aus der Lösung durch Lyophilisation oder Sprühtrocknung; wobei das Aztreonam-lysinat zu einer Teilchengröße von 1 bis 5 Mikron gemahlen, gefällt, sprühgetrocknet oder anderweitig verarbeitet wird.

2. Verfahren nach Anspruch 1, wobei das Mahlen durch Mahlen mit einem Mahlmedium zu einem Pulver mit einem Massemedian des mittleren Durchmessers von 1 bis 5 Mikron erreicht wird.

3. Verfahren nach Anspruch 1, wobei das Mahlen durch Strahlmahlen zu einem Pulver mit einem Massemedian des mittleren Durchmessers von 1 bis 5 Mikron erreicht wird.

4. Verfahren nach Anspruch 1, wobei das Trocknen des Aztreonamlysinats durch Sprühtrocknen erreicht wird.

5. Verfahren nach Anspruch 1, wobei die Teilchengröße durch Teilchenfällungstechniken zu einem Pulver mit einem Massemedian des mittleren Durchmessers von 1 bis 5 Mikron erreicht wird.

6. Verfahren nach Anspruch 1, wobei das Trocknen des Aztreonamlysinats durch Lyophilisation erreicht wird.

7. Verfahren nach Anspruch 1, wobei die solubilisierte Aztreonam-Zusammensetzung bei einem pH-Wert unter 6 gehalten wird.

8. Verfahren nach Anspruch 1, durchgeführt bei einer Temperatur von 2 bis 20 °C.

9. Verfahren nach Anspruch 8, durchgeführt bei einer Temperatur von 2 bis 8 °C.

10. Verfahren nach Anspruch 7, wobei die solubilisierte Aztreonam-Zusammensetzung auf einem pH-Wert von 4,6 bis 4,8 gehalten wird.

11. Verfahren nach einem der Ansprüche 1 bis 10, bei dem ein Reservoir der trockenen Aztreonam-lysinat-Pulverzubereitung in eine Vorrichtung gegeben wird, die zur direkten Abgabe des trockenen Pulvers in die Lunge geeignet ist.

12. Verfahren nach einem der Ansprüche 1 bis 11, bei dem eine Einzeldosis der trockenen, inhalierbaren Aztreonam-lysinat-Pulverzubereitung in einem Behälter zubereitet wird.

## Revendications

1. Procédé de préparation d'une formulation de lysinate d'aztréonam en poudre sèche inhalable constituée de lysinate d'aztréonam, ledit procédé comprenant les étapes :
a) de solubilisation de la forme alpha de l'aztréonam dans l'eau pour former une suspension épaisse ;
b) de titrage d'une solution aqueuse de lysine pour former du lysinate d'aztréonam en solution ; et
c) de séchage du lysinate d'aztréonam à partir de la solution par lyophilisation ou par séchage par pulvérisation ; et dans lequel le lysinate d'aztréonam est broyé, précipité, séché par pulvérisation ou traité d'une autre manière pour obtenir une taille de particule entre 1 et 5 microns.

2. Procédé selon la revendication 1 dans lequel le broyage est réalisé en réduisant le milieu en une poudre ayant un diamètre moyen moyen en masse de 1 à 5 microns.

3. Procédé selon la revendication 1 dans lequel le broyage est réalisé par jet jusqu'à obtention d'une poudre ayant un diamètre moyen moyen en masse de 1 à 5 microns.

4. Procédé selon la revendication 1 dans lequel le séchage du lysinate d'aztréonam est réalisé par séchage par pulvérisation.

5. Procédé selon la revendication 1 dans lequel la taille de particule est obtenue par des techniques de précipitation de particules jusqu'à obtention d'une poudre ayant un diamètre moyen moyen en masse de 1 à 5 microns.

6. Procédé selon la revendication 1 dans lequel le séchage du lysinate d'aztréonam est réalisé par lyophilisation.

7. Procédé selon la revendication 1 dans lequel la composition de lysinate d'aztréonam solubilisée est maintenue à un pH inférieur à 6.

8. Procédé selon la revendication 1 réalisé à une température de 2 à 20°C.

9. Procédé selon la revendication 8 réalisé à une température de 2 à 8°C.

10. Procédé selon la revendication 7 dans lequel la composition d'aztréonam solubilisée est maintenue à un pH entre 4,6 et 4,8.

11. Procédé selon l'une quelconque des revendications 1 à 10 dans lequel un réservoir de la formulation de lysinate d'aztréonam en poudre sèche est placé dans un dispositif utilisable pour administrer la poudre sèche directement dans les poumons.

12. Procédé selon l'une quelconque des revendications 1 à 11 dans lequel une dose individuelle de la formulation de lysinate d'aztréonam en poudre sèche inhalable est fabriquée dans un récipient.
